(19) Europäisches Patentamt
European Patent Office
Office européen des brevets

(11) EP 0 930 361 B1

(12) EUROPEAN PATENT SPECIFICATION

(45) Date of publication and mention of the grant of the patent:
**11.06.2003 Bulletin 2003/24**

(21) Application number: **97928515.2**

(22) Date of filing: **30.06.1997**

(51) Int Cl.[7]: **C12N 5/00**, C12N 5/06

(86) International application number:
**PCT/JP97/02254**

(87) International publication number:
**WO 98/001537 (15.01.1998 Gazette 1998/02)**

(54) **CELL COMPOSITIONS**

ZELLZUSAMMENSTELLUNGEN

COMPOSITIONS CELLULAIRES

(84) Designated Contracting States:
**DE FR GB IT NL**

(30) Priority: **10.07.1996 JP 18050096**

(43) Date of publication of application:
**21.07.1999 Bulletin 1999/29**

(73) Proprietor: **TAKARA BIO INC.**
**Otsu-shi, Shiga (JP)**

(72) Inventors:
- **ASADA, Kiyozo**
  **Koka-gun, Shiga 520-33 (JP)**
- **KONISHI, Haruko**
  **Kyoto-shi, Kyoto 601 (JP)**
- **KOYAMA, Nobuto**
  **Uji-shi, Kyoto 611 (JP)**
- **KATO, Ikunoshin**
  **Uji-shi, Kyoto 611 (JP)**

(74) Representative: **Vossius, Volker, Dr. et al**
**Dr. Volker Vossius,**
**Patentanwaltskanzlei - Rechtsanwaltskanzlei,**
**Geibelstrasse 6**
**81679 München (DE)**

(56) References cited:
**EP-A- 0 642 793** **EP-A- 0 795 560**
**WO-A-95/26200** **WO-A-96/16973**

- **KYALHEIM G. ET AL: "Triggering of the APO-1/FAS antigen (CD95) selectively kills leukemic cells without compromising hematopoietic stem cells: a novel strategy for tumor cell purging of autografts" BLOOD, vol. 84, no. 10, 1994, page 570A XP002130860**
- **EUROPEAN JOURNAL OF CANCER & CLINICAL ONCOLOGY, (1989), Vol. 25(2), BRON D., STRYCKMAN P., "Removal of Tumor Cells from Bone Marrow Overview", p. 163-166.**
- **BLOOD, (1994), Vol. 84, 10, Supplement 1, GUNNAR KYALHEIM et al., "Triggering of APO-1/FAS Antigen (CD95) Selectively Kills Leukamic Cells Without Compromising Hematopoietic Stem Cells", pp. 570A.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 930 361 B1

Printed by Jouve, 75001 PARIS (FR)

**Description**

FIELD OF THE INVENTION

The present invention relates to in vitro processes for obtaining cell compositions comprising hematopoietic stem cells from which cancer cells have been substantially elim-nated.

BACKGROUND OF THE INVENTION

As progress in cell biology, physiological properties and functions of various cells have been revealed and methods utilizing cells per se for medical purposes have been developed. For example, although chemotherapy and radiotherapy have been employed for treating cancers, sometimes, such therapeutic methods cause lethal damages to normal cells, in particular, bone marrow cells. Then, in order to reduce such a risk, autologous bone marrow transplantation, in which bone marrow cells harvested from a patient prior to a treatment is returned to the patient after the treatment, has been proposed. Further, transformation techniques of target cells by transferring required exogenous genes into the target cells have recently been developed as gene therapy. For example, when a multiple drug resistant gene is transferred into bone marrow cells, the cells acquire multiple drug resistance, thereby making it possible to treat cancers with drugs which have not been heretofore employed in such treatment because of their severe cytotoxic activity on bone marrow cells. Blood, vol 84, 1994, 570A discloses the selective willing of leukemic cells in hematopoietic stem cell compositions with an anti - CD95 (Apo-I/EAS) antibody.

Thus, compositions containing suitable cells, or compositions containing suitably modified cells are of very importance in the medical field. However, in case that target cell compositions are contaminated with cancer cells, sufficient therapeutic effect cannot be obtained. In particular, when the above multiple drug resistant gene is transferred into a cell composition contaminated with cancer cells, the contaminated cancer cells also acquire multiple drug resistance, which adversely affects on the required cancer treatment.

One problem of the present invention is to provide a process for obtaining a cell composition comprising hematopoietic stem cells in which cancer cells have been substantially eliminated.

SUMMARY OF THE INVENTION

The present inventors have found that only cancer cells contaminating a hematopoietic stem cell composition can be specifically eliminated by using an apoptosis inducer which specifically induces apoptosis of cancer cells, and that gene therapy can be carried out in safety by transferring a required exogenous gene into the hematopoietic stem cells after the treatment with the apoptosis inducer. The solution of this problem is indicated in claims 1 to 5.

That is, the first aspect of the present invention is a process for obtaining a cell composition comprising hematopoietic stem cells substantially free from cancer cells, in particular, the cell composition comprising hematopoietic stem cells from which cancer cells have been substantially eliminated by using an apoptosis inducer specific to the cancer cells. The representative example of the composition is a buffer solution comprising such hematopoietic stem cells.

The amount of the hematopoietic stem cells in the composition is not specifically limited and can be determined according to the particular use of the composition. In addition, the buffer solution may contain other hematopoietic cells, stromal cells and the like, and it may contain a culture substrate for the hematopoietic stem cells, a hematopoietic stem cell growth factor, a stem cell differentiation factor, a hematopoietic stem cell protecting agent and the like.

The second aspect of the present invention is a method for obtaining a cell composition comprising hematopoietic stem cells substantially free from cancer cells and, in particular, the method comprising a step for selectively eliminating cancer cells by using an apoptosis inducer specific to the cancer cells. In a further step the hematopoietic stem cells may be transfected with an exogenous gene.

Apoptosis is one mode of cell deaths which is different from necrosis. From morphological viewpoint, it takes place through nucleus condensation, cell shrinkage, vacuolation, cell surface smoothing, cell fragmentation and the like, and is a representative mode of programmed cell deaths (Nikkei Biotech Ed., Nikkei Biotechnology New Terminology Dictionary, 4th Ed., p 21-22).

According to the present invention, by using the apoptosis inducer, the required hematopoietic stem cells into which an exogenous gene has been transferred can be grafted into a host without such a risk that the gene is transferred into cancer cells.

DETAILED DESCRIPTION OF THE INVENTION

The apoptosis inducers used in the process of the present invention are sulfated polysaccharides or their

decomposition products having apoptosis inducing properties; those containing saccharide compounds containing uronic acids and/or uronic acid derivatives or their decomposition products having apoptosis inducing properties and those containing 4,5-dihydroxy-2-cyclopenten-1-one represented by the formula (1):

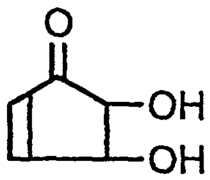

(1)

The apoptosis inducer alone or in combination thereof with a known pharmaceutically acceptable carrier can be formulated into a pharmaceutical preparation according to a per se known method. The pharmaceutically acceptable carrier can be appropriately selected according to a particular form of the preparation and, for example, in case of a solid preparation, lactose, sucrose, mannitol, starch, carboxymethylcellulose, inorganic salts or the like can be used. Contact between cells and the above apoptosis inducer can be conducted by adding the above preparation to the cell composition or dissolving it in the cell composition.

Examples of the sulfated polysaccharides used in the present invention include fucoidans, dextran sulfates and the like.

Fucoidans are polysaccharides containing fucose sulfates in their molecules. Although it is not limited to a specific one, fucoidans are contained, for example, in brown algae, sea cucumber and the like [Supervisor: Tokuro Souda, Editor: Fujio Egami, "Tatorui-Kagaku (Polysaccharide Chemistry)" published Dec. 15, 1955 by Kyoritsu Shuppan K.K., pages 319 and 321]. It has been known that fucose sulfate-containing polysaccharides derived from brown algae are generally called as fucoidans, fucoidins and fucans and there are some molecular species. In the present specification, all of them are included in fucoidans. In addition, in the present invention, decomposition products of fucoidans can also be used.

As fucoidans used in the present invention, fucoidan-containing extracts obtained from fucoidan-containing materials, or purified materials obtained from the extracts can be used. The preparation of fucoidan-containing extracts and the purification of the extracts can be carried out according to per se known methods and they are not limited to a specific one.

Further, decomposition products of fucoidans are those obtained by decomposing fucoidans chemo-enzymatically, chemically or physically and any known chemo-enzymatic, chemical or physical method can be employed.

As brown algae containing fucoidans, there are, for example, those described by Yukio Yamada and Sokichi Segawa, Colored Illustrations of the Seaweeds of Japan, published by HOIKUSHA, 1977, pages 22 to 52 and, for example, fucoidans can be prepared by using *Fucus evanescens, Kjellmaniella crassifolia, Laminaria japonica,* and *Undaria pinnatifida*, among others.

As sea cucumber containing fucoidans, for example, there are those described in JP-A 4-91027 and, for example, *Stichopus japonicus*, and *Holothuria lecospilota* can be used. Fucoidans can be prepared by the method described therein.

Fucoidan-containing powders can be prepared by drying brown algae, sea cucumber, etc. and then pulverizing the dried materials.

Further, fucoidan-containing extracts can be prepared by extracting the fucoidan-containing powders with hot water or diluted acids.

As the purification means of the extracts for increasing the fucoidan content, there are fractionation of fucoidans with calcium chloride, barium acetate, etc.; fractionation of fucoidans with acidic polysaccharide aggregation agents such as cetylpyridinium chloride, etc.; fractionation of fucoidans with acidic polysaccharide aggregation agents in the presence of bases; gel filtration; ion exchange chromatography; and the like. The purification can be carried out by combining these purification methods when necessary.

As the decomposition method of fucoidans, know methods for decomposing fucoidans such as those using fucoidan decomposing enzymes, acid decomposition, sonication and the like can be employed. The decomposition products can be purified by the above methods.

Fucoidans have sulfate groups in their molecules and such groups react with various bases to form salts. Fucoidans and their decomposition products in the form of salts are more stable than those having free sulfate groups and, normally, they are isolated in the form of salts such as those with sodium and/or potassium, etc. These salts can be led to fucoidans and their decomposition products having free sulfate groups by treating the salts with cation exchange resins such as Dowex 50, etc. Further, the salts can be replaced by various other desired salts by known conventional salt replacement when necessary. As salts of fucoidans and their decomposition products, pharmaceutically acceptable salts are used and examples thereof include salts with alkali metals such as potassium, sodium, etc., salts with alkaline earth metals such as calcium, magnesium, barium, etc., salts with organic bases such as pyridinium, ammonium salt and the like.

Among molecular species of fucoidans, there are two groups, one contains fucose as the main component, and the other contains several % of uronic acids and relatively larger amounts of fucose and mannose as constituent sugars. Hereinafter, fucoidans substantially free from uronic acids are called as Fucoidan-F and fucoidans containing uronic acids are called as Fucoidan-U. A mixture thereof is simply indicated as Fucoidan.

In the present invention, Fucoidan-F and Fucoidan-U can be used alone respectively, or in combination thereof.

Namely, the apoptosis inducer used in the present invention may contain, for example, Fucoidan-U having the following physicochemical properties which is prepared according to the method as described in Example 1 hereinafter.

(1) Constituent sugars: it is mainly composed of fucose, mannose and galactose, and containing uronic acids.
(2) It is decomposed to low molecular products with a fucoidan decomposing enzyme produced by *Flavobacterium* sp. SA-0082 (FERM BP-5402).

Alternatively, the apoptosis inducer used in the present invention may contains, for example, Fucoidan-F having the following physicochemical properties which is prepared according to the method as described in Example 2 hereinafter.

(1) Constituent sugars: it is mainly composed of fucose and substantially free from uronic acids.
(2) No substantial decomposition to low molecular products is caused with a fucoidan decomposing enzyme produced by *Flavobacterium* sp. SA-0082 (FERM BP-5402).

Microbial decomposition products of fucoidans can be prepared by treating fucoidans with microorganisms capable of decomposing fucoidans, for example, the above *Flavobacterium* sp. SA-0082 which produces the fucoidan decomposing enzyme. Furthermore, enzymatic decomposition products of Fucoidan-U can be prepared by treating the above Fucoidan-U with fucoidan decomposing enzymes, for example, the above fucoidan decomposing enzyme produced by *Flavobacterium* sp. SA-0082. In addition, fractionated products can be prepared from these decomposition products according to their molecular weight, respectively.

In general, fucoidans are liable to be affected by acids and alkalis. Then, when acidic or alkaline solutions are used, they can be readily decomposed to low molecular weight products. By adjusting heating temperature, time, pH and the like, any desired decomposition products can be prepared and, for example, Average molecular weight, molecular weight distribution and the like of decomposition products can be adjusted by gel filtration treatment, molecular weight-fractionation membrane treatment and the like. In addition, molecular weight and sugar compositions of fucoidans are varied depending upon the harvest season of raw materials, and methods for drying and storing the raw materials as well as extraction conditions of fucoidans such as heating conditions, pH conditions and the like. For example, fucoidans are hydrolyzed by acids. On the other hand, under alkaline conditions, formation of low molecular weight products proceeds due to β-elimination of uronic acids. Therefore, as for Fucoidan-U and Fucoidan-F described herein, their molecular weight and molecular weight distribution are mere exemplification, and their molecular weight and molecular weight distribution can be readily varied according to the conditions treating fucoidans. For example, when heating is carried out at 100°C for 1 hour and a molecular sieve membrane having the pore size 300 is used for desalting, Fucoidan, Fucoidan-U or Fucoidan-F having the molecular weight distribution of about 1,000 to 10,000 can be prepared. Fucoidans having any molecular weight and molecular weight distribution can be prepared according to the conditions employed and, in the present invention, the apoptosis inducers containing these fucoidans can be used.

When fucoidans or their decomposition products are added to a culture broth of cancer cells at a concentration of 1 μg/ml or more, apoptosis of the cancer cells is caused within one to several days after addition, thereby showing the strong apoptosis inducing activity of fucoidans or their decomposition products. On the other hand, these products do not induce any apoptosis of normal cells and do not show any toxicity against normal cells. In particular, fucoidans and their decomposition products derived from edible brown algae and sea cucumber are very safe because they are derived from naturally occurring materials and no toxicity is observed by oral administration to mouse.

For using fucoidans as the apoptosis inducer, fucoidans and/or their decomposition products may be combined with known pharmaceutically acceptable carriers to obtain pharmaceutical preparations and the pharmaceutical preparations may be allowed to come into contact with the desired cells.

Dextran sulfates are the sulfates of dextrans which are polymers of D-glucopyranoses attached through α-1,6-bonds and produced by microorganisms, for example *Leuconostoc mesenteroides*. In the present invention, commercially available dextran sulfates can be used.

When dextran sulfates or their heat-treated products are added to a culture broth of cancer cells, apoptosis of the cancer cells is caused within one to several days after addition, thereby showing the apoptosis inducing activity of dextran sulfates or their decomposition products. For using dextran sulfates as the apoptosis inducer, they may be combined with known pharmaceutically acceptable carriers to obtain pharmaceutical preparations and the pharmaceutical compositions may be allowed to come into contact with the desired cells.

In addition, the saccharide compounds containing uronic acids and/or uronic acid derivatives are those selected from polysaccharides, oligosaccharides and monosaccharides containing uronic acids and/or uronic acid derivatives in the molecules and are not specifically limited to a specific one in so far as they have apoptosis inducing activity.

Examples of polysaccharides containing uronic acids and/or uronic acid derivatives include pectin, pectic acid, alginic acid, hyaluronic acid and the like.

Examples of oligosaccharides containing uronic acids and/or uronic acid derivatives include oligosaccharides derived from the above polysaccharides and they can be produced according to known methods. Further, oligosaccharides synthesized by synthetic methods are also included in the present invention.

Examples of uronic acids and uronic acid derivatives include galacturonic acid, glucuronic acid, mannuronic acid, their lactones, their esters, for example methyl esters and their amides, and they can be produced by known methods.

The saccharide compounds to be used in the present invention which contain uronic acids and/or uronic acid derivatives, and have apoptosis inducing activity can be produced by using saccharide compounds containing uronic acids and/or uronic acid derivatives as raw materials. Although the origins of raw materials and production methods are not limited to a specific one, they can be produced, for example, by using polysaccharides containing uronic acids and/or uronic acid derivatives as constituent components, for example, pectin and pectic acid. Further, in the production of the saccharide compounds, although their production methods are not limited to a specific one, they can be produced from raw materials, for example, by chemical, enzymatic or physical production methods alone or in combination thereof.

As the chemical treatment for the production of the saccharide compounds to be used in the present invention, for example, a raw material is treated at a room temperature to 200°C for several seconds to several hours, preferably, 50 to 130°C for several seconds to 60 minutes. In case of pectin, for example, β-elimination reaction is caused by treatment under conditions at pH 6.8 at 95°C for several seconds to several minutes to obtain a saccharide compound containing unsaturated uronic acids and/or unsaturated uronic acid derivatives whose absorbance at about 235 nm is increased. The saccharide compounds of the present invention include those having unsaturated uronic acids and/or uronic acid derivatives at their non-reducing ends formed by β-elimination reaction of polysaccharides containing uronic acids and/or uronic acid derivatives.

The decomposition products of the saccharide compounds to be used in the process of the present invention containing uronic acids and/or uronic acid derivatives are obtained by enzymatic hydrolysis, e.g. with lyases. For instance, in case of pectin and pectic acid, the saccharide compounds to be used in the present invention can be obtained by decomposing them with a known pectin lyase (EC4. 2. 2. 10), pectic acid lyase (EC4. 2. 2. 2), and exopolygalacturonic acid lyase (EC4. 2. 2. 9), respectively, to form the saccharide compounds having 4-deoxy-L-threo-hexa-4-enopyranosyl uronate or their methyl esters at the non-reducing ends. Further, hyaluronic acid lyase (EC4. 2. 2. 1) is used in case of hyaluronic acid, and alginic acid lyase (EC4. 2. 2. 3) is used in case of alginic acid.

As the physical treatment for the production of the saccharide compounds to be used in the present invention, examples thereof include treatment with near infrared rays, infrared rays, microwaves, ultrasonic waves and the like. For example, pectin and/or pectic acid are added to a solution at a neutral or alkaline pH and subjected to sonication to give vibrational energy at an appropriate temperature above a room temperature for 1 second or more, preferably for 5 seconds to 1 hour under appropriate reducing conditions, for example, in the presence of ascorbic acid. As described above, in addition to ultrasonic waves, irradiation of microwaves, near infrared rays and infrared rays are also effective and they can be irradiated in combination. Irradiation can be carried out continuously or intermittently.

The decomposition products of saccharide compounds containing uronic acids and/or uronic acid derivatives and having apoptosis inducing activity to be used in the present invention can be produced by using the saccharide compounds containing uronic acids and/or uronic acid derivatives as raw materials. Although production methods of the decomposition products are not limited to specific one, for example, they are produced from raw materials by chemical, enzymatic and physical production methods or in combination thereof.

Examples of the above decomposition products to be used include heat-treated products of saccharide compounds containing uronic acids and/or uronic acid derivatives.

As heat treatment methods in the production of the above heat-treated products, for example, the saccharide compounds are subjected to heat treatment at, for example, 65 to 350°C for several seconds to several days, preferably 80 to 150°C for several minutes to several days to obtain the heat-treated products having apoptosis inducing activity.

Pectin to be used as the saccharide compounds is not specifically limited and, for example, high molecular weight polysaccharides extracted from the peel of citrus fruits and the fruits of apple. Raw materials for the industrial

production of pectin are fruits and, in addition to lees remained after squeezing juice from citrus fruits such as lemon, lime and the like (mainly inner skins), lees remained after obtaining juice from apple can be used. These lees mainly contain proto-pectin and the latter is solubilized (extracted) during production steps to prepare pectin. Solubilization can be carried out by extracting with acidic warm or hot water, and pectin having stable molecular weight and esterification degrees can be obtained in high yield by controlling extraction temperature, pH and time conditions according to raw materials used. The extract can be purified by centrifugation or filtration and concentrated and alcohol is added thereto whereupon pectin can be precipitated and recovered. The precipitate can be dried and pulverized to prepare the desired dried pectin.

The main structure of pectin is a polymer of partially methylated galacturonic acids. The carboxyl group may be esterified with methyl group or may be free. Or, the carboxyl group may form a ammonium salt, potassium salt or sodium salt. According to its esterification degree with a methyl group (DM degree: the ratio of methoxy groups to total carboxyl groups), pectin is divided into HM pectin having a high DM degree and LM pectin having a low DM degree [Tomoshi Yoshizumi et al., Ed., Shin-Shokuhin Kaihatuyou Sozai Binran (Handbook of New Materials for Development of Food), p 114-119, published by Korin Shoten (1991)]. In the present invention, commercially available pectin as a food additive [Akio Tonoyama Ed., Tennenbutsu Binran (Handbook of Natural Occurring Substances), 12th Ed., p 138, published by Shokuhin to Kgaku Sha (1993)], commercially available HM pectin, commercially available LM pectin (see above Handbook of New Materials for Development of Food) and the like can be used.

In the process of the present invention, decomposition products of pectin and pectic acid can be used. As decomposition methods of pectin, there are, for example, chemical decomposition methods such as acid treatment, alkaline treatment and the like, physical decomposition methods such as sonication, heat treatment, pressure treatment, pressure and heat treatment and the like, or enzymatic decomposition methods.

The saccharide compounds containing uronic acids and/or uronic acid derivatives, or their decomposition products having apoptosis inducing properties to be used in the present invention include pharmaceutically acceptable salts thereof.

For using as the apoptosis inducer, the saccharide compounds containing uronic acids and/or uronic acid derivatives, or their decomposition products having apoptosis inducing properties can be combined with known pharmaceutically acceptable carriers to prepare pharmaceutical preparations.

When the saccharide compounds containing uronic acids and/or uronic acid derivatives, or their decomposition products having apoptosis inducing properties are added to a culture broth of cancer cells, apoptosis of the cancer cells is caused one to several days after addition. Further, the apoptosis inducers do not show any toxicity against normal cells.

The saccharide compounds containing uronic acids and/or uronic acid derivative or their decomposition products having apoptosis inducing properties are derived from naturally occurring materials and no toxicity is observed upon oral and parenteral administration thereof to mice.

The 4,5-dihydroxy-2-cyclopenten-1-one having apoptosis inducing activity to be used in the process of the present invention and represented by the formula (1) (hereinafter simply referred to as cyclopentenone) can be synthesized by chemical synthesis [Carbohydrate Res., 247, 217-222 (1993); and Helvetica Chimica Acta, 55, 2838-2844 (1972)] and both trans- and cis-isomers can be used in the present invention.

Further, when an aqueous solution of D-glucuronic acid is subjected to heat treatment at 121°C for 4 hours, the cyclopentenone can be formed in the heat-treated material. The cyclopentenone in the heat-treated material is extracted with a solvent and the extract is concentrated. Then, the concentrate is fractionated by silica gel column chromatography, the cyclopentenone fraction eluted is concentrated and then the cyclopentenone is extracted from the concentrate with chloroform. The extract is concentrated and subjected to normal phase column chromatography to obtain the purified cyclopentenone.

The physical properties of the cyclopentenone are as follows. In the following properties, mass analysis was carried out by using DX 302 mass spectrometer (manufactured by Nippon Denshi). The NMR spectrum using deuterated chloroform solvent is determined by using JNM-A500 (manufactured by Nippon Denshi). The specific rotatory power is determined by using DIP-370 polarimeter (manufactured by Nippon Bunko), the UV absorption spectrum is determined by using UV-2500 spectrophotometer (manufactured by Shimadzu) and the IR absorption spectrum is determined by using FTIR-8000 infrared spectrophotometer (manufactured by Shimadzu), respectively.

MS m/z 115 $[M+H]^+$

$^1$H-NMR($CDCl_3$) δ4.20(1H, d, J=2.4 Hz, H-5), 4.83 (1H, m, H-4), 6.30 (1H, dd, J=1.2, 6.1 Hz, H-2), 7.48 (1H, dd, J=2.1, 6.1 Hz, H-3), provided that the chemical shift value of $^1$H-NMR was shown by taking the chemical shift value of $CHCl_3$ as 7.26 ppm.

Specific rotatory power: $[\alpha]_D^{20}$ 0° (c=1.3, $H_2O$)
IR (KBr) : 3400, 1715, 1630, 1115, 1060, 1025 $cm^{-1}$
UV: λmax 215 nm ($H_2O$)

For using the cyclopentenone as the apoptosis inducer, it can be combined with a known pharmaceutically acceptable carrier to obtain a pharmaceutical preparation.

When the cyclopentenone is added to a culture broth of cancer cells, apoptosis of the cancer cells are caused within one to several days after addition. Further, it does not show any toxicity against normal cells.

The cyclopentenone used in the present invention does not show any toxicity against mouse.

Hematopoietic stem cells are those having differentiation ability to matured blood cells such as erythrocytes, granulocytes, platelets, lymphocytes, etc. from a single cell (pluripotency) and also having self replication capability.

Hematopoietic stem cells are utilized for (1) renewal of the hematopoietic system of a hematopoietic stem cell-deficient host, (2) treatment by re-transplantation hematopoietic stem cells prepared from bone marrow, which has been harvested from a host with a disease prior to administration of a drug, irradiation, etc., into the host after the treatment (3) production of various hematopoietic cells, and (4) treatment of diseases by gene transfer into autologous hematopoietic stem cells, among others.

For obtaining hematopoietic stem cells, it is necessary to isolate and prepare pluripotent hematopoietic stem cells from the cell population of bone marrow or other hematopoietic sources. First, bone marrow cells can be obtained from a bone marrow source, for example, crista iliace, tibia, femur, vertebra or other bone cavities. Other sources of hematopoietic stem cells include fetal liver, fetal and adult spleen and blood such as adult peripheral blood and umbilical cord blood.

As a method for isolation of hematopoietic stem cells and for preparing their compositions, although any known method can be used, at present, a simplest and most efficient method for preparing hematopoietic stem cell compositions is that disclosed by JP-A 7-313150 and substantially uniform compositions of human hematopoietic stem cells can be prepared by that method. For example, a fraction containing the desired cells is obtained by magnetic separation using magnetic beads covered by an antibody, affinity chromatography, using a monoclonal antibody, etc., and the fraction is further separated by a fluorescence-activated cell sorter to obtain the desired cells. The cells are suspended in a buffer culture medium.

The composition containing the isolated hematopoietic stem cells can be used according to particular purposes. However, if the composition is contaminated with cancer cells, various problems may be caused depending upon their uses.

Thus, the present inventors have succeeded in elimination of only cancer cells present in a hematopoietic stem cell composition by using an apoptosis inducer specific to cancer cells.

The apoptosis inducer in the present invention can be used in concentration sufficient to induce apoptosis of cancer cells and to eliminate only the cancer cells. The apoptosis inducer can be simply added to a culture broth of hematopoietic stem cells. Further, it can be added in any suitable step during preparation of the hematopoietic stem cell composition. However, the most efficient results can be obtained by adding the apoptosis inducer to the hematopoietic stem cell composition immediately after its preparation.

The cell composition of the present invention is that containing hematopoietic stem cells thus obtained, and can be produced by a per se known method. The amount of hematopoietic stem cells in the composition is not specifically limited and can be determined by a particular use of the composition. In addition, the composition can contain other hematopoietic cells, stromal cells and the like and it can further contain a hematopoietic stem cell culture substrate, a hematopoietic stem cell growth factor, a stem cell differentiation factor, a hematopoietic stem cell protecting agent and the like.

According to the process of the present invention, the hematopoietic stem cell composition in which cancer cells are to be substantially eliminated, can be obtained even from peripheral blood of a patient with multiple myeloma which is considered, at present, to be most difficult to obtain a cancer cell-free hematopoietic stem cell composition therefrom [Blood, 86, 381-389 (1995)].

The hematopoietic stem cell composition prepared can be proliferated by known methods, for example, the method described in the above JP-A 7-313150. For example, it can be proliferated by co-culture with stromal cells, in a culture medium containing a maintenance factor, or the like.

Hematopoietic stem cells can be used as target cells of gene transfer. Although a gene can be transferred into target cells according to known methods, as a method for transferring a gene into target cells such as hematopoietic stem cells, the most efficient method is that disclosed in WO 95/26200.

Genes which are transferred into target cells, i.e. exogenous genes may be any genes which are desired to be transferred into cells. For example, the exogenous genes may be those encoding proteins relating to diseases such as adenosine deaminase (ADA), antisense nucleic acids or ribozymes, or false primers (see, for example, WO 90/13641 published November 15, 1990), intracellular antibodies (see, for example, WO 94/02610 published February 3, 1994),

growth factors and the like.

These exogenous genes can be transferred into target cells under the control of promoters suitable for controlling expression of these genes, typically, exogenous promoters. Further, expression controlling factors other than promoters, for example, terminator sequences and enhancer sequences can be added, when necessary.

Gene transfer into hematopoietic stem cells can be carried out by using, for example, retroviral vectors. The vectors can contain marker genes such as antibiotic resistant genes so that cells into which the desired gene has been transferred can be readily selected. Examples of the representative vectors to be used in the present invention include N2/ZipTKNEO (TKNEO) vector (titer: $1 \times 10^5$ G418r cfu/ml on NIH 3T3 cells), ZipPGK-hADA vector, ZipPGK-mADA vector and the like. All of them have been reported by Moritz et al., J. Exp. Med., 178, 529 (1993).

TKNEO vector has neomycin phosphotransferase gene expressed by the herpes simplex thymidine kinase promoter. Cells into which the desired gene has been transferred by using this vector can be selected by utilizing neomycin resistance provided by the marker gene. In ZipPG K-hADA vector, human ADA (hADA) cDNA is expressed by human phosphoglycerate kinase (PGK) promoter. This gene is only one expressible gene of the vector and the vector does not have any selectable marker gene. ZipPGK-mADA (PGK-mADA) vector is the same as ZipPGK-hADA vector except that human ADA cDNA is replaced with mouse ADA (mADA) cDNA. Properties and production processes of these vectors and other viral vectors are well known and their selection and use will be well within those skilled in the art given the disclosure herein.

The composition of hematopoietic stem cells into which the desired gene has been transferred can be used for treatment of genetic diseases. Hematopoietic cell-relating genetic diseases can be treated by grafting the composition of autologous or allogenic hematopoietic stem cells having the gene that can make up for the deficiency or the abnormality of the gene causing the diseases.

For example, since genes which cause such diseases as β-thalasemia (Mediterranean anemia), sickle cell anemia, ADA deficiency, recombinase deficiency, recombinase regulatory gene deficiency and the like have been identified, treatment can be carried out by transferring a normal wild type gene into the gene of hematopoietic stem cells by homologous or random recombination and grafting the cells into a patient.

Further, in case of allogenic hematopoietic stem cells, normal hematopoietic stem cells free from abnormalities of genes can be used for treatment.

Another application of gene therapy is to make possible to use a drug in high concentration, which is normally considered to be danger, by providing drug resistance to normal hematopoietic stem cells by transferring a drug resistant gene into the cells. In particular, it is possible to carry out the treatment using an anticancer drug in high concentration by transferring a gene having drug resistance against the anticancer drug, e.g., a multiple drug resistant gene into the hematopoietic stem cell composition substantially free from cancer cells obtained according to the present invention.

Even for diseases other than those relating to the hematopoietic system, the diseases can be treated by using the hematopoietic stem cells in so far as the diseases relate to deficiency of secretory proteins such as hormones, enzymes, cytokines, growth factors and the like. A deficient protein can be induced and expressed by transferring a gene encoding the protein in question into the hematopoietic stem cells under the control of a suitable promoter. Even if the protein is expressed by cells whose cell type is different from that for the normal expression of the protein, the expression can be controlled so that the same activity as that obtained by the natural expression thereof is obtained.

In addition to complementing deficiency and abnormality of a gene as described above, it is possible to insert a gene encoding a ribozyme, an antisense nucleic acid or the like or another suitable gene into cells to control expression of a specific gene product in the cells or to inhibit liability to diseases, in particular, hematic diseases.

For example, for hematic pathogens such as HIV, HTLV-I, HTLV-II and the like, hematopoietic stem cells can be subjected to gene modification to express an antisense nucleic acid or a ribozyme, which can prevent growth of the above pathogens, in hematopoietic stem cells or cells differentiated from hematopoietic stem cells.

Alternatively, among cell surface receptors, a specific molecule can be eliminated from cells belonging to T cells. Namely, expression of a specific receptor can be inhibited by using gene modification of the receptor gene by homologous recombination, or by using an antisense nucleic acid or a ribozyme which inhibits expression of the receptor gene.

The hematopoietic stem cell composition thus obtained into which a gene has been transferred can be introduced in a vertebrate, which is a recipient of cell grafting, by, for example, conventional intravenous administration. Although the recipient is preferably a donor per se, allogenic grafting can be carried out. In particular, when umbilical cord blood cells are used in the grafting, the latter is preferred.

By using the apoptosis inducer having cancer cell selectivity of the present invention, cancer cells in the composition of target cells for gene transfer can be selectively eliminated. The target cells are not specifically limited and examples thereof include cells selected from stem cells, hematopoietic cells, primordial germ cells, oocytes, oogonia, ova, spermatocytes, sperms, CD34+cells, C-kit+cells, lymphocytes, B cells, T cells, bone marrow cells and the like. The compositions containing these cells can be prepared according a known method, respectively.

Further, when embryonic stem cells, primordial germ cells, oocytes, oogona, ova, spermatocytes, sperms and the like are used as the target cells, transformant vertebrates can be simply and readily created.

The following examples further illustrate the present invention in detail but are not to be construed to limit the scope thereof. In the following example, all %'s are by weight.

Example 1

(1) After thoroughly drying *Kjellmaniella crassifolia*, the alga (2 kg) was pulverized by a free pulverizer M-2 (manufactured by Nara Kikai Seisakusho) and the dried powder was suspended in 80% ethanol (9 liters). The suspension was treated at 80°C for 2 hours and then filtered through a filter paper to obtain a residue. The residue was subjected to the same procedure of ethanol washing and filtration treatment three times, repeatedly, to obtain a residue washed with ethanol. The residue was suspended in 0.2 M calcium acetate solution (36 liters). The suspension was treated at 95°C for 2 hours and filtered. The resultant residue was washed with 0.2 M calcium acetate solution (4 liters) and the washing was combined with the above filtrate to obtain a fucoidan extract of *Kjellmaniella crassifolia* (36 liters).

The filtrate was concentrated to 2 liters by an ultrafilter apparatus equipped with an ultrafilter membrane having exclusion molecular weight of 0.1 million. To this was added sodium chloride at the final concentration of 1.5 M and then added 5% cetylpyridinium chloride in such an amount that no more precipitate was formed. The precipitate formed was removed by centrifugation. The resultant supernatant was concentrated to 1 liter by ultrafiltration and ethanol (4 liters) was added thereto. The resultant precipitate was collected by centrifugation. To this precipitate was added 4 M sodium chloride (100 ml) and, after thoroughly stirring, ethanol was added at the final concentration of 80%. The mixture was stirred and centrifuged to obtain a precipitate. The resultant precipitate was subjected to the same procedure of suspending in 80% ethanol and centrifugation, repeatedly, until the absorbance at 260 nm in the supernatant was disappeared. The precipitate was dissolved in 2 M sodium chloride (2 liters) and insoluble materials were removed by centrifugation. Then, to the resultant solution was added DEAE-Cellulofine A-800 (manufactured by Seikagaku Kogyo, 50 ml) and the mixture was stirred, followed by removing the resin added by filtration. The filtrate was applied to a DEAE-Cellulofine A-800 column equilibrated by 2 M sodium chloride and the unadsorbed fraction was subjected to ultrafiltration with an ultrafilter apparatus equipped with a hollow fiber having exclusion molecular weight of 0.1 million or less to completely remove coloring materials and sodium chloride. Then, insoluble materials were removed by centrifugation and filtration and the filtrate was lyophilized to prepare Fucoidan-U. The amount of the dried Fucoidan-U was 15 g.

Molecular weight of the resultant Fucoidan-U was determined by gel filtration using Sephacryl S-500. It showed molecular weight distribution having the median value of about 0.19 million.

(2) Fig. 1 shows precipitate formation properties of the above Fucoidan-U and Fucoidan-F prepared in Example 2 hereinafter at different sodium chloride concentrations in the presence of an excess amount of cetylpyridinium chloride.

In Fig. 1, the vertical axis represents a precipitate formation rate (%) and the horizontal axis represents concentration of sodium chloride (M). In Fig. 1, the solid line and blank circles represent the precipitate rates of Fucoidan-U at respective sodium chloride concentrations. In Fig. 1, the dotted line and blank triangles represent the precipitate formation rates of Fucoidan-F at different sodium chloride concentrations (M).

The precipitate formation rate was determined at a solution temperature of 37°C as follows.

Fucoidan-U and Fucoidan-F were dissolved in water and 4 M sodium chloride at a concentration of 2%, respectively, and the resultant solutions were mixed in various proportions so as to prepare solutions (each 125 μl) of Fucoidan-U and Fucoidan-F having different sodium chloride concentrations. On the other hand, cetylpyridinium chloride was dissolved in water and 4 M sodium chloride at a concentration of 2.5%, respectively and they were mixed so as to prepare 1.25% cetylpyridinium chloride solutions having different sodium chloride concentrations.

For completely precipitating Fucoidan-U and Fucoidan-F from their 2% solutions in water with 1.25% solution of cetylpyridinium chloride, 3.2-fold as much cetylpyridinum chloride solution as the fucoidan solution by volume was required. Then, 400 μl of cetylpyridinium chloride solutions of respective sodium chloride concentrations were added to 125 μl of 2% Fucoidan-U and Fucoidan-F solutions of respective sodium chloride concentrations, and the resultant mixtures were thoroughly stirred. The mixtures were allowed to stand for 30 minutes and then centrifuged. The saccharide content in the supernatants was determined by phenol-sulfuric acid method [Analytical Chemistry, 28, 350 (1956)] to calculate the precipitate formation rate of the fucoidans at respective sodium chloride concentrations.

(3) The components of the above Fucoidan-U were analyzed by the following methods.

First, the amount of fucose was determined according to the description of Journal of Biological Chemistry, 175, 595 (1948).

The dry standard of Fucoidan-U obtained was dissolved in 1 N hydrochloride acid at a concentration of 0.5% and hydrolyzed into constituent monosaccharides by treating at 110°C for 2 hours. The reducing ends of the monosaccharides obtained by hydrolysis were pyridyl-(2)-amination (PA derivative) with GlycoTAG™ and GlycoTAG™ Reagent

Kit (both manufactured by Takara Shuzo) and subjected to HPLC to determine the proportion of the constituent sugars.

Next, uronic acids were determined according to the description of Analytical Biochemistry, 4, 330 (1962).

Further, sulfuric acid content was determined according to the description of Biochemical Journal, 84, 106 (1962).

As a result, the constituent sugars of Fucoidan-U were fucose, mannose, galactose, glucose, rhamnose, xylose and uronic acids.

Any other neutral saccharide was not contained. The molar ratio of the main components fucose : mannose : galactose : uronic acids : sulfate group was about 10 : 7 : 4 : 5 : 20.

(4) The structure of Fucoidan-U was determined as follows.

(4)-1 The following endo type fucoidan decomposing enzyme was reacted with purified Fucoidan-U and the resultant decomposition product was purified.

Namely, 1% Fucoidan-U solution (16 ml), 50 mM phosphate buffer (pH 8.0, 12 ml), 4 M sodium chloride solution (4 ml) and a solution of the following endo type fucoidan decomposition enzyme (32 mU/ml, 8 ml) were mixed and the mixture was reacted at 25°C for 48 hours. As the reaction proceeded, an increase in the absorbance at 230 nm was recognized, indicating that the Fucoidan-U was decomposed by that enzyme.

After desalting of the reaction mixture with Microancilyzer G3 (manufactured by Asahi Kasei), the reaction mixture was separated into three fractions (a), (b) and (c) and purified by DEAE Sepharose FF (manufactured by Pharmacia).

The endo type fucoidan decomposing enzyme can be prepared as follows.

A microbial strain to be used for producing the endo type fucoidan decomposing enzyme may be any strain which is capable of producing the endo type fucoidan decomposition enzyme. Examples thereof include *Flavobacterium* sp. SA-0082 strain (FERM BP-5402).

This strain was investigated and newly obtained from sea water in Aomori, Japan and this strain was indicated as *Flavobacterium* sp. SA-0082 and has been deposited with National Institute of Bioscience and Human-Technology, Agency of Industrial Science and Technology at 1-3, Higashi 1 chome, Tsukuba-shi, Ibaraki-ken, Japan according to the Budapest Treaty under the accession number of FERM BP-5402 since March 29, 1995 (the date of the original deposit).

Any nutrient source may be added to the culture medium of this strain in so far as it is assimilable by the strain to produce the endo type fucoidan decomposing enzyme. Examples of carbon sources include, fucoidans, powdered algae, alginic acid, fucose, glucose, mannitol, glycerol, sucrose, maltose, lactose, starch and the like. Suitable examples of nitrogen sources include yeast extract, peptone, Casamino Acids, corn steep liquor, meat extract, defatted soybeans, ammonium sulfate, ammonium chloride and the like. In addition, inorganic substances such as sodium salts, phosphate salts, potassium salts, magnesium salts, zinc salts and the like and metal salts can also be added.

For culturing the producer of the endo type fucoidan decomposition enzyme, although yield is varied depending upon culture conditions, in general, an incubation temperature at 15°C to 30°C and pH of the culture medium at 5 to 9 are preferred, and yield of the endo type fucoidan decomposing enzyme reaches the maximum by aeration-agitation culture for 5 to 72 hours. Naturally, culture conditions are set so as to obtain the maximum yield of the endo type fucoidan decomposing enzyme.

The endo type fucoidan decomposing enzyme is present in both microbial cells and culture supernatant.

The above *Flavobacterium* sp. SA-0082 strain is cultured in a suitable culture medium, and their cells are collected and disrupted by a conventional cell disruption means, for example, ultrasonication to obtain a cell free extract.

Then, a purified enzyme standard can be obtained from this extract by a conventional purification means. For example, purification can be carried out by salting-out, ion exchange column chromatography, hydrophobic bond column chromatography, gel filtration column chromatography and the like to obtain the purified endo type fucoidan decomposing enzyme free from other fucoidan decomposing enzymes.

Further, since a large amount of the enzyme (extracellular enzyme) is present in a culture supernatant obtained by removing cells from the above culture broth, it can be purified by the same manner as that for the intracellular enzyme. An embodiment of purification of the endo type fucoidan decomposing enzyme is as follows.

A culture medium (600 ml) composed of artificial sea water containing glucose (0.25%), peptone (1.0%) and yeast extract (0.05%) (manufactured by Jamarine Laboratory, pH 7.5) had been distributed into a 2-liter Erlenmeyer flask, followed by sterilization (120°C for 20 minutes). *Flavobacterium* sp. SA-0082 (FERM BO-5402) was inoculated into the culture medium (600 ml) in the 2-liter Erlenmeyer flask and incubated at 24°C for 24 hours to obtain a seed culture broth. A culture medium (20 liters) composed of artificial sea water containing glucose (0.25%), peptone (1.0%), yeast extract (0.05%) and a defoamer (manufactured by Shinetsu Chemical, 0.01%) (manufactured by Jamarine Laboratory, pH 7.5) was placed in a 30-liter jar fermenter and sterilized at 120°C for 20 minutes. After cooling, the above seed culture broth (600 ml) was inoculated and incubated at 24°C for 24 hours under culture conditions of 10 liters/minute of aeration with agitation at 125 r.p.m. After culture was completed, the culture broth was centrifuged to obtain microbial cells.

The cells were suspended in 20 mM acetate-phosphate buffer (pH 7.5) containing 200 mM sodium chloride, followed by ultrasonication and then centrifugation to obtain a cell extract. When the activity of the endo type fucoidan decomposing enzyme of the present invention in the cell extract was determined, the activity of 5 mU/ml culture broth was detected. The determination of the activity will be illustrated hereinafter.

To this extract was added ammonium sulfate at a final concentration of 90% saturation and the mixture was stirred to dissolve the ammonium sulfate. The mixture was centrifuged and the resultant precipitate was suspended in the same buffer as that of the above microbial cell extract. The suspension was sufficiently dialyzed against 20 mM acetate-phosphate buffer (pH 7.5) containing 50 mM sodium chloride. The precipitate resulted from the dialysis was removed by centrifugation and then the supernatant was applied on a DEAE Sepharose FF column equilibrated with 20 mM acetate-phosphate buffer (pH 7.5) containing 50 mM sodium chloride. The material absorbed by the column was thoroughly washed with the same buffer and the column was eluted with a linear concentration gradient of 50 mM to 600 mM sodium chloride to collect an active fraction. Then, sodium chloride was added to the active fraction at a final concentration of 4 M. The fraction was applied on a Phenyl Sepharose CL-4B (manufactured by Pharmacia) column equilibrated with 20 mM phosphate buffer (pH 8.0) containing 4 M sodium chloride. The adsorbate was thoroughly washed with the same buffer and the adsorbate was eluted with a linear concentration gradient of 4 M to 1 M sodium chloride to collect an active fraction. Then, the active fraction was concentrated with an ultrafilter and the concentrate was subjected to gel filtration column chromatography with Sephacryl S-300 (manufactured by Pharmacia) equilibrated with 10 mM phosphate buffer containing 50 mM sodium chloride to collect an active fraction. When molecular weight of the enzyme was determined from the retention time on Sephacryl S-300, it was about 0.46 million. Further, the active fraction was dialyzed against 10 mM phosphate buffer (pH 7) containing 25 mM sodium chloride. This solution of the enzyme was applied on a Mono Q HR5/5 (manufactured by Pharmacia) column equilibrated with 10 mM phosphate buffer (pH 7) containing 250 mM sodium chloride. The adsorbent was thoroughly washed with the same buffer and the column was eluted with a linear concentration gradient of 250 mM to 450 mM sodium chloride to collect an active fraction. Thus, the purified enzyme was obtained. These production steps are shown in Table 1. Determination of protein is carried out by measuring the absorbance of the enzyme solution at 280 nm. In this determination, the absorbance of a solution of 1 mg/ml of protein is taken as 1.0.

Table 1

| Step | Total amount of protein (mg) | Total activity (mU) | Specific activity (mU/mg) | Yield (%) |
|---|---|---|---|---|
| Cell extract | 61,900 | 101,000 | 1.63 | 100 |
| Salting-out with ammonium sulfate | 33,800 | 88,600 | 2.62 | 87.7 |
| DEAE Sepharose FF | 2,190 | 40,400 | 18.4 | 40.0 |
| Phenyl Sepharose CL-4B | 48.2 | 29,000 | 601 | 28.7 |
| Sephacryl S-300 | 7.24 | 19,600 | 2,710 | 19.4 |
| MonoQ | 0.824 | 15,000 | 18,200 | 14.9 |

The activity of the enzyme is determined as follows.

A 2.5% solution of a fucoidan derived from *Kjellmaniella crassifolia* (50 µl), the enzyme solution (10 µl) and 83 mM phosphate buffer (pH 7.5) containing 667 mM sodium chloride (60 µl) are mixed and allowed to react with each other at 37°C for 3 hours. Then, the enzymatic reaction mixture (105 µl) is mixed with water (2 ml). The mixture is stirred and its absorbance at 230 nm (AT) is measured. As controls, the reactions are carried out under the same conditions by using only the above buffer used for dissolving the enzyme instead of the enzyme solution and by using only water instead of the fucoidan solution and the absorbance of the reaction mixtures are measured (AB1 and AB2).

The amount of the enzyme required for eliminative cleavage of 1 µmol of the glycoside bond between mannose and a uronic acid per 1 minute is defined as 1 unit (U) of the enzyme. The determination of cleaved bonds is carried out by taking the mmol molar absorbancy index of unsaturated uronic acids formed by the elimination reaction as 5.5 to calculate the bonds. The enzyme activity is calculated by the following equation:

$$(AT-AB1-AB2)\times 2.105\times 120/5\times 105\times 0.01\times 180=U/ml$$

wherein 2.105 is the amount of the sample solution whose absorbance is measured (ml); 120 is the amount of the

enzyme reaction mixture (μl); 5.5 is mmol molar absorption coefficient of unsaturated uronic acids at 230 nm (/mM); 105 is the amount of the reaction mixture to be diluted (μl); 0.01 is the amount of the enzyme solution (ml); and 180 is the reaction time (minutes).

The fucoidan derived from *Kjellmaniella crassifolia* used as the substrate was prepared as followed.

Dried *Kjellmaniella crassifolia* is pulverized with a free pulverizer M-2, followed by treatment in 10-fold volume of 85% methanol at 70°C for 2 hours and filtration. The resultant residue is treated in 10-fold volume of methanol at 70°C for 2 hours, followed by filtration. To the residue is added 20-fold volume of water and the mixture is treated at 100°C for 3 hours and filtered to obtain an extract. The salt concentration of the extract is adjusted to the same as that of 400 mM sodium chloride solution. Then, cetylpyridinium chloride is added thereto until no more precipitate is formed, and the mixture is centrifuged. The precipitate is thoroughly washed with ethanol and, after completely removing cetylpyridinium chloride, desalting and removal of low molecular weight materials are carried out by an ultrafilter apparatus (exclusion molecular weight of the ultrafilter membrane: 0.1 million, manufactured by Amicon). The precipitate formed is removed by centrifugation. The supernatant is lyophilized to obtain the purified *Kjellmaniella crassifolia* fucoidan.

(4)-2 The above endo type fucoidan decomposing enzyme cleaves α 1→4 bond between D-mannose and D-glucuronic acid present in complex polysaccharides by an elimination reaction. When it is reacted with Fucoidan, oligosaccharides having the structures represented by the following formulas (2), (3) and (4) are formed.

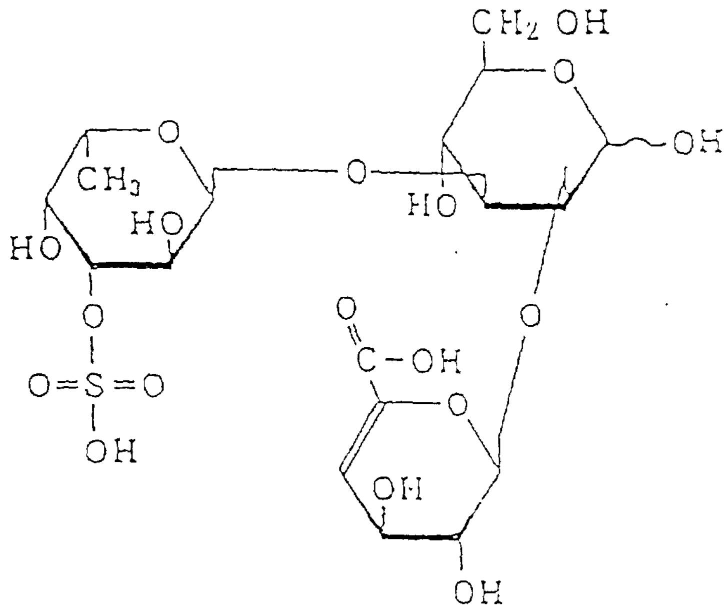

(2)

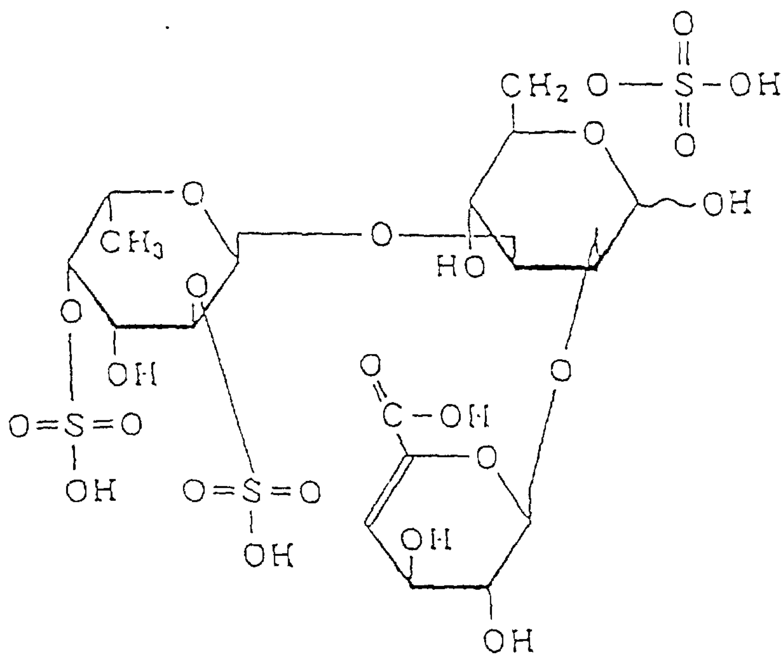

(3)

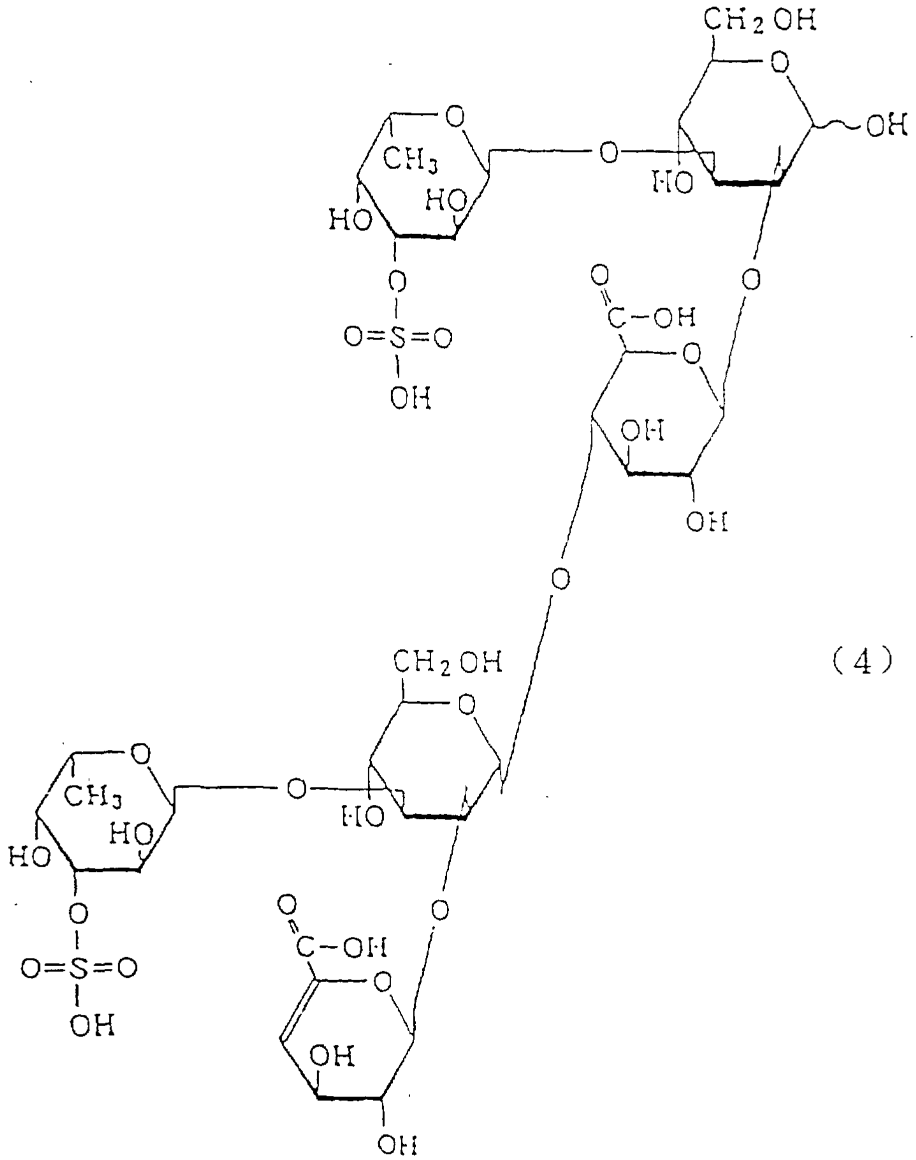

(4)

Then, respective portions of the above three fractions (a), (b) and (c) separated and purified with DEAE Sepharose FF were subjected to pyridyl-(2)-amination of their reducing ends (PA derivative) with GlycoTAG and GlycoTAG Reagent Kit to obtain PA derivatives of saccharides (PA-a), (PA-b) and (PA-c), respectively. (PA-a), (PA-b) and (PA-c) were analyzed by HPLC to examine the difference between them and the PA derivatives of the three oligosaccharides having the structures represented by the above formulas (2), (3) and (4).

HPLC conditions were as follows.

(i) HPLC analysis using molecular weight fraction column

Apparatus: L-6200 (manufactured by Hitachi Seisakusho)
Column: SHODEX SB-803 (4.6×250 mm) (manufactured by Showa Denko)
Eluting solution: 0.2 M sodium chloride dimethyl sulfoxide = 9 : 1
Detection: a fluorescent detector F-1150 (manufactured by Hitachi Seisakusho) with the excitation wavelength of 320 nm; detection is carried out at the fluorescent wavelength of 400 nm.
Flow rate: 1 ml/min.
Column temperature: 50°C

(ii) HPLC analysis using reverse phase column

Apparatus: L-6200 (manufactured by Hitachi Seisakusho)
Column: L-column (4.6x250 mm) (manufactured by Kagaku Yakuhin Kensa Kyokai)
Eluting solution: 50 mM acetic acid-triethylamine (pH 5.5)
Detection: a fluorescent detector F-1150 (manufactured by Hitachi Seisakusho) with the excitation wavelength of 320 nm; detection is carried out at the fluorescent wavelength of 400 nm.
Flow rate: 1 ml/min.
Column temperature: 40°C

As a result of the above two HPLC analyses, it was found that three oligosaccharides obtained by decomposing Fucoidan-U with the above endo type fucoidan decomposing enzyme were the same as the three olygosaccharides having the structures represented by the above formulas (2), (3) and (4).

Therefore, (a) has such structure that both unsaturated D-glucuronic acid and L-fucose, to which sulfate group is attached, are attached to the reducing end residue, D-mannose; (b) has such structure that both unsaturated D-glucuronic acid and L-fucose, to which two sulfate groups are attached, are attached to the reducing end residue, D-mannose; and (c) has such structure that D-both glucuronic acid and L-fucose, to which sulfate group is attached, are attached to the reducing end residue, D-mannose, in addition, another D-mannose is attached to said D-glucuronic acid, and both unsaturated D-glucuronic acid and L-fucose, to which sulfate group is attached, are further attached to the latter D-mannose.

In view of tne above, the resultant Fucoidan-U has such structure that D-glucuronic acids and D-mannoses are alternately attached to each other, and L-fucose is attached to at least one D-mannose.

Further, it has the partial structure represented by the formula:

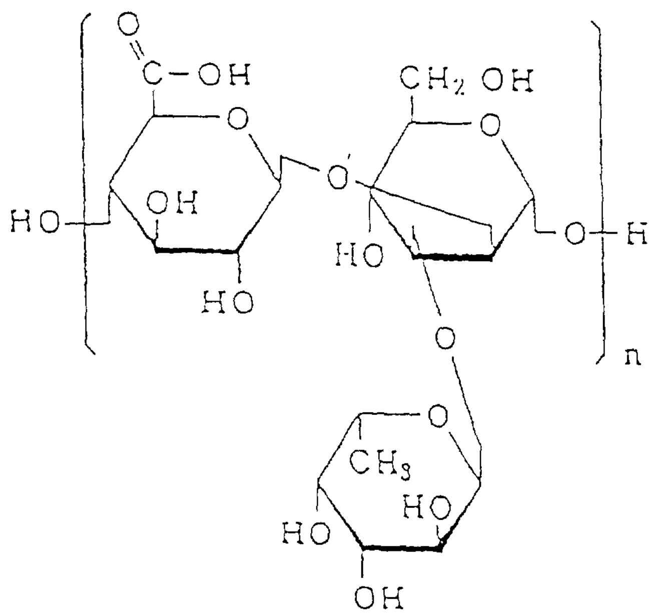

(5)

wherein at least one alcoholic hydroxide group is esterified with sulfuric acid and n is an integer of 1 or more.

As describe hereinabove, when Fucoidan-U was reacted with the above endo type fucoidan decomposing enzyme, the oligosaccharides represented by the above structural formulas (2), (3) and (4) were formed.

When the specific rotatory power of a lyophilized product of Fucoidan-U was measured by a high speed, high sensitive polarimeter SEPA-300 (manufactured by Horiba Seisakusho), it was -53.6°.

Example 2

(1) *Kjellmaniella crassifolia* was thoroughly dried and a portion thereof (2 kg) was pulverized with a free pulverizer M-2. The resultant dried powder was suspended in 80% ethanol (9 liters) and treated at 80°C for 2 hours,

followed by filtration with a filter paper to obtain a residue. The residue was subjected to the same procedure of washing with ethanol and filtration three times, repeatedly, to obtain a residue washed with ethanol. The residue was suspended in 0.2 M calcium acetate (36 liters) and treated at 95°C for 2 hours, followed by filtration. The residue was washed with 0.2 M calcium acetate (4 liters) and the washing was combined with the above filtrate to obtain a *Kjellmaniella crassifolia* fucoidan extract (36 liters).

To the resultant extract was added 5% cetylpyridinium chloride until no more precipitate was formed and the precipitate was collected by centrifugation. The precipitate was suspended in 0.4 M sodium chloride, centrifuged and washed. The washing procedures were repeated 3 times and 4 M sodium chloride (1 liter) was added to the precipitate. The mixture was stirred and ethanol was added at a final concentration of 80%. The resultant mixture was stirred and centrifuged to obtain a precipitate. The precipitate was suspended in 80% ethanol and centrifuged. The procedures were repeated until the absorbance at 260 nm was disappeared. The precipitate was dissolved in 2 M sodium chloride (3 liter) and insoluble materials were removed by centrifugation. Then, DEAE-Cellulofine A-800 (100 ml) was added thereto and the mixture was stirred, followed by filtration to remove the resin added. The filtrate was applied to a DEAE-Cellulofine A-800 column equilibrated with 2 M sodium chloride and an unadsorbed portion was subjected to ultrafiltration by using an ultrafilter apparatus equipped with a hollow fiber having exclusion molecular weight of 0.1 million or less to completely removing coloring materials and sodium chloride, followed by centrifugation and filtration to remove insoluble materials and lyophilization to obtain a fucoidan.

The amount of the lyophilized fucoidan was 90 g.

The lyophilized fucoidan (7 g) was weighed and dissolved in 0.2 M calcium chloride. Then, a DEAE Sepharose FF column was equilibrated with 0.2 M calcium chloride. The fucose sulfate-containing polysaccharide mixture dissolved in 0.2 M calcium chloride was applied on the DEAE Sepharose FF column and thoroughly washed with 0.2 M calcium chloride and eluted with a linear concentration gradient of 0 to 4 M sodium chloride. Among fractions eluted, the fractions eluted by sodium chloride ranging in concentration from 0.05 to 0.8 M were collected and desalted by dialysis, followed by lyophilization to obtain Fucoidan-U (2.1 g) substantially free from Fucoidan-F.

Further, among the above fractions, the fractions eluted by sodium chloride ranging in concentration from 0.9 to 1.5 M were collected and desalted by dialysis, followed by lyophilization to obtain Fucoidan-F (4.7 g) substantially free from Fucoidan-U.

When molecular weight of the above Fucoidan-F was determined by gel filtration column chromatography using Sephacryl S-500, it showed molecular weight distribution having the median value of about 0.19 million.

(2) The components of Fucoidan-F were analyzed according to the method described in Example 1.

The constituent sugars of this Fucoidan-F were fucose and galactose and its molar ratio was about 10 : 1. No substantive uronic acid and other neutral saccharides were contained. In addition, the molar ratio of fucose and sulfate group was about 1 : 2.

A 1% solution of Fucoidan-F (16 ml) was mixed with 50 mM phosphate buffer (pH 8.0, 12 ml), 4 M sodium chloride (4 ml) and a solution of the endo type fucoidan decomposing enzyme described in Example 1-(3) (32 mU/ml, 8 ml) and the mixture was reacted at 25°C for 48 hours. No formation of decomposition products due to the reaction was observed.

When a specific rotatory power of the lyophilized product of this Fucoidan-F was determined by a high speed, high sensitive polarimeter SEPA-300 (manufactured by Horiba Seisakusho), it was -135°.

Example 3

(1) Myeloma cells (P3X63Ag8, ATCC TIB-9) were cultured at 37°C in RPMI1640 culture medium (manufactured by Gibco) containing 10% fetal bovine serum (manufactured by JRH Bioscience) treated at 56°C for 30 minutes. The cells were suspended in RPMI1640 culture medium containing 10% fetal bovine serum at a concentration of $1 \times 10^4$ cells/1.8 ml. On the other hand, Fucoidan-U described in Example 1 and Fucoidan-F described in Example 2 were dissolved in 50 mM HEPES buffer (pH 7.2) containing 100 mM sodium chloride and treated by heating at 120°C for 20 minutes, respectively. Each of these solutions (0.2 ml) was added to the above cell suspension (1.8 ml) at a fucoidan concentration of 5, 10 or 20 mg/ml and the mixture was cultured at 37°C for 92 hours in the presence of 5% $CO_2$.

The cultured cells were observed under a microscope to examine the degree of growth and morphology of cells. As a result, myeloma cells to which Fucoidan-U or Fucoidan-F had been added exhibited characteristics of apoptosis such as cell shrinkage, cell nucleus fragmentation, and the like. The myeloma cell number of a control group to which no sample had been added increased up to about 70-fold, while myeloma cells to which Fucoidan-U or Fucoidan-F had been added were killed, indicating that these two fucoidans showed strong apoptosis inducing activity. The living cell number was counted with time after initiation of incubation according to the method described in Sosiki Baiyou no Gizyutsu (Technique for Tissue Culture, Vol. 2, The Society of Japan Tissue Culture Ed. published by Asakura Shoten, pages 26-28, 1990. Namely, the number of cells which were stained by trypan blue was counted on a hemocytometer to obtain the living cell number.

The results are shown in Figs. 2 and 3. Figs. 2 and 3 are graphs illustrating the relation between the incubation time and the living cell number. The horizontal axis represents the incubation time and the vertical axis represents the living cell number in a culture broth. Fig. 3 is an enlarged graph of Fig. 2, wherein the scale on the vertical axis is enlarged. In Figs. 2 and 3, × represents the control (C) without addition of any sample, the blank circle represents the addition of Fucoidan-U at a concentration of 0.5 mg/ml, the blank triangle represents the addition of Fucoidan-U at a concentration of 1 mg/ml, the blank square represents the addition of Fucoidan-U at a concentration of 2 mg/ml, the filled circle represents of the addition of Fucoidan-F at a concentration of 0.5 mg/ml, the filled triangle represents of the addition of Fucoidan-F at a concentration of 1 mg/ml and the filled square represents the addition of Fucoidan-F at a concentration of 2 mg/ml.

(2) Human acute myelocytic leukemia cells HL-60 (ATCC CCL-240) were cultured at 37°C in RPMI1640 culture medium (manufactured by Gibco) containing 10% fetal bovine serum (manufactured by JRH Bioscience) treated at 56°C for 30 minutes. The cells were suspended in ASF104 culture medium (manufactured by Ajinomoto) at a concentration of $5 \times 10^4$ cells/900 μl. Each 4.5 ml portion of the suspension was distributed in each well of a 6-well plate manufactured by FALCON. On the other hand, each of Fucoidan-U described in Example 1 and Fucoidan-F described in Example 2 was dissolved in 30 mM HEPES buffer (pH 7) containing 120 mM sodium chloride at a concentration of 10 mg/ml and filtrated through a filter. Each of these filtrates (0.5 ml) was added to the above suspension and incubated at 37°C in the presence of 5% $CO_2$. As a control, the same amount of the above buffer was added and the mixture was incubated according to the same manner. The living cell number was counted at 16 hours and 40 hours after initiation of incubation according to the same manner as described above.

The results are shown in Fig. 4. Namely, Fig. 4 is a graph illustrating the relation between the incubation time and the living cell number obtained by adding Fucoidan-U or Fucoidan-F to the HL-60 cell culture broth at a concentration of 1 mg/ml. The horizontal axis represents the incubation time and the vertical axis represents the living cell number in the culture broth. In Fig. 4, the blank circle represents Fucoidan-U and the filled circle represents Fucoidan-F. The living cell number of the control (without addition of the sample) was $7 \times 10^4$ cells/ml at 16 hours after initiation of the incubation and $1.4 \times 10^5$ cells/ml at 40 hours after initiation of the incubation.

As a result, it has been found that apoptosis of HL-60 cells has been caused by Fucoidan-U and -F to inhibit their cell growth rate.

In addition, Fucoidan-U and Fucoidan-F were dissolved in 30 mM HEPES buffer (pH 7) containing 120 mM sodium chloride at a concentration of 10 mg/ml, respectively, and autoclaved at 121°C for 20 minutes. Apoptosis inducing activity of these solutions were determined according to the same manner as described above and the same results were obtained.

(3) 5-Fluorouracil (5-FU, manufactured by Amresco, 150 mg/kg) was administered to 6 to 8-week old mice (C3H/HeJ), intraperitoneally. After 2 days, the femurs and tibias were excised to collect bone marrow. The resultant bone marrow was subjected to density gradient centrifugation by using Ficoll Hypaque (density 1.0875 g/ml, manufactured by Pharmacia) to prepare a low density mononuclear cell fraction. This was served as mouse bone marrow cells.

The mouse bone marrow cells were subjected to liquid culture in the presence or in the absence of Fucoidan-U described in Example 1 or Fucoidan-F described in Example 2. Namely, the above mouse bone marrow cells at a cell density of $1 \times 10^6$ cells/ml were added to α-MEM (manufactured by Gibco) containing 20% fetal bovine serum, recombinant human Interleukin-6 (rhIL-6, 100 U/ml, manufactured by Amgen), recombinant mouse stem cell factor (rmSCF, 100 ng/ml, manufactured by Amgen), penicillin (50 U/ml) and streptomycin (50 μg/ml), and further Fucoidan-U or Fuccidan-F (1 mg/ml) was added thereto. Then, the mixture was incubated at 37°C for 48 hours in 5% $CO_2$.

After completion of incubation, non adhered cells were collected by decantation and cells adhered to a plate used for incubation were collected by using a cell dissociation buffer (CDB, without any enzyme, manufactured by Gibco). The cells were combined and the cell number was counted. The collected cells were subjected to HPP-CFC (High Proliferative Potential-Colony Forming Cells) assay.

HPP-CFC assay was carried out according to the method described by Bradley et al. [Aust. J. Exp. Biol. Med. Sci., 44, 287-293 (1966)]. As the culture medium, 1%/0.66% overlayed soft agar culture medium was used and the infected cells were added at a concentration of $5 \times 10^4$ cells/well. The cells were incubated at 37°C for 13 days in 10% $CO_2$. After completion of incubation, the colonies appeared were observed under an inverted microscope and high density colonies derived from HPP-CFC (0.5 mm or more diameter) were counted.

The results are shown in Fig. 5. Namely, Fig. 5 is a graph illustrating the relation between the fucoidan and the high density colony number. The horizontal axis represents the fucoidan used and the control without addition of any fucoidan and the vertical axis represents the high density colony number. As seen from Fig. 5, regarding the high density colony number, no significant difference was recognized between the addition of Fucoidan-U or -F to the culture medium and the control.

Example 4

Myeloma cells (P3X63Ag8U.1, ATCC CRL-1597) were cultured at 37°C in RPMI1640 culture medium (manufactured by Gibco) containing 10% fetal bovine serum (manufactured by JRH Bioscience) treated at 56°C for 30 minutes and they were suspended in RPMI1640 culture medium containing 10% fetal bovine serum at a concentration of $2.5 \times 10^5$ cells/4.5 ml. On the other hand, a dextran sulfate (molecular weight: 0.5 million, manufactured by Oncor) was dissolved in 50 mM HEPES buffer (pH 7.0) containing 120 mM sodium chloride at a concentration of 10 mg/ml and sterilized by filtration. This solution (0.5 ml) was added to the above suspension (4.5 ml) and incubated at 37°C for 60 hours in the presence of 5% $CO_2$.

The incubated cells were observed under a microscope to examine the degree of growth and cell 1 morphology. As a result, myeloma cells to which the dextran sulfate had been added exhibited characteristics of apoptosis such as cell shrinkage, cell nuclear fragmentation and the like. The cell number of the control myeloma cell to which any sample had not been added was increased up to about 20-fold, while the myeloma cells to which the dextran sulfate was added were killed. Thus, the dextran sulfate exhibited strong apoptosis inducing activity. After initiation of incubation, the living cell number was counted with time by trypan blue staining.

The result is shown in Fig. 6. Fig. 6 is a graph illustrating the relation between the incubation time and the living cell number. The horizontal axis represents the incubation time and the vertical axis represents the living cell number in the culture broth. In Fig. 6, the blank square represents the control without addition of any sample and the blank circle represents the addition of the dextran sulfate (1 mg/ml).

Next, A dextran sulfate (molecular weight: 0.5 million, manufactured by Oncor) was dissolved in 50 mM HEPES buffer (pH 7.0) containing 120 mM sodium chloride in concentration of 10 mg/ml, and treated by heating at 120°C for 20 minutes. According to the same manner as described above, apoptosis inducing activity was determined by using the dextran sulfate.

The results are shown in Fig. 7. Fig. 7 illustrates the relation between the incubation time and the living cell number. The horizontal axis represents the incubation time and the vertical axis represents the living cell number in the culture broth. In Fig. 7, the blank square represents the control without addition of any sample and the filled circle represents the addition of heat-treated dextran sulfate (1 mg/ml). The heat-treated dextran sulfate has also strong apoptosis inducing activity.

Example 5

Commercially available lemon pectin was dissolved in 50 mM HEPES buffer (pH 7.0) containing 120 mM sodium chloride at a concentration of 10 mg/ml. The pH of the solution was 5.0. It was treated by heating at 121°C for 30 minutes. When UV absorption spectrum was measured, the absorbance at about 235 nm of the heat-treated material was increased.

These samples were adjusted to pH 7.0 with 1 N sodium hydroxide and, according to the same manner as described in Example 3-(2), apoptosis inducing activity was determined.

The results are shown in Fig. 8. The heat-treated pectin exhibited remarkable apoptosis inducing activity. Namely, Fig. 8 is the graph illustrating the incubation time and the living cell number in a culture broth, when a solution of the heat-treated pectin (1 mg/ml) was added to the HL-60 cell culture broth. The horizontal axis represents the incubation time and the vertical axis represents the living cell number in the culture broth. In Fig. 8, the blank square represents the control without addition of any sample and the blank lozenge represents the addition of the heat-treated pectin.

Example 6

(1) D-glucuronic acid (10 g, manufactured by Sigma G 5269) was dissolved in water (1 liter), heated at 121°C for 4 hours, and concentrated to about 10 ml under reduced pressure. To this was added an upper layer (40 ml) of the mixture of butyl acetate-acetic acid-water (3 : 2 : 2) and the mixture was stirred and centrifuged. The supernatant was concentrated to about 10 ml under reduced pressure.

The above extract was subjected to column chromatography with silica gel BW-300SP column ($2 \times 28$ cm, manufactured by Fuji Silicia Chemical) and fractionation was carried out by using an upper layer of butyl acetate-acetic acid-water (3 : 2 : 2) as the eluting solution at the flow rate of 5 ml/minute under pressure of 0.2 kg/$cm^2$ with a compressor. Each 10 ml fraction was collected and a portion thereof was analyzed by TLC. As a result, the fraction Nos. 61 to 80 contained the cyclopentenone in high purity. These fractions were combined and concentrated under reduced pressure and then extracted with chloroform (about 40 ml). The extract was concentrated under reduced pressure to obtain the cyclopentenone (about 100 mg).

The fraction was subjected to normal phase HPLC using PALPAK Type S column (manufactured by Takara

Shuzo) and detected by UV absorption at 215 nm. As a result, the purity was 98%.

(2) The umbilical cord blood vessel endothelial cells, HUVEC cells (primary culture, manufactured by Clonetics, CC-2517), was passaged once and frozen for storage according to a conventional manner. The cells were thawed and, after washing twice with phosphate buffered saline (manufactured by Takara Shuzo), they were suspended in RPMI1640 culture medium containing 10% fetal bovine serum at a concentration of $1 \times 10^5$ cells/ml. Each 90 μl portion of the cell suspension was distributed to each well of a 96-well microtiter plate and added thereto 10 μl of aqueous 10, 20, 50, 100, 200, 500 or 1,000 μM cyclopentenone solution or water (control). The cells on that plate were incubated at 37°C for 48 hours in the presence of 5% $CO_2$. Then, a solution (10 ul) of 3-(4,5-dimethylthiazol-2-yl)-2,5-diphenylretrazolium bromide (MTT, manufactured by Sigma) in phosphate buffered saline (5 mg/ml) was added thereto and the incubation was continued for additional 4 hours. Then, growth of the cells were observed under a microscope. Further, 2-propanol containing 0.04 N HCl (100 μl) was added, the mixture was thoroughly stirred and the absorbance at 590 nm was measured. The ratio of the absorbance at 590 nm of the cyclopentenone added group to the absorbance at 590 nm of the control group, wherein the incubation was carried out by addition of only water, was calculated to determine apoptosis inducing activity in terms of cell growth inhibitory activity.

The same procedure was carried out with respect to HL-60 cells except that the cells used were passaged in RPMI1640 culture medium containing 10% fetal bovine serum. As a result, the cyclopentenone had stronger cell growth inhibitory activity to the cancer cells, HL-60 cells than that to the normal cells, HUVEC cells.

The results are shown in Fig. 9. Fig. 9 is the graph illustrating the relation between the amount of cyclopentenone added (final concentration) and the degree of cell growth. In Fig. 9, the horizontal axis represents the cyclopentenone concentration (final concentration, μM) and the vertical axis represents the ratio (%) of the absorbance at 590 nm of the cyclopentenone added group to the absorbance at 590 nm of the water added group. In Fig. 9, the blank circle represents the results obtained by using HUVEC and the filled circle represents the results obtained by using HL-60. Further, the cell growth observed under a microscope was in parallel with the absorbance at 590 nm.

(3) The fibroblasts, NIH/3T3 cells (ATCC CRL-1658), were dispersed with trypsin according to a conventional method and suspended in Dulbecco modified Eagle's culture medium containing 10% bovine serum at a concentration of $5 \times 10^4$ cells/ml. Then, each 90 μl portion thereof was distributed into each well of a 96-well microtiter plate. To each well was added an aqueous solution (10 μl) of 15.6, 31.3, 62.5, 125, 250, 500 or 1,000 μM cyclopentenone, or water (10 μl) as a control and the cells on that plate were incubated at 37°C for 48 hours in the presence of 5% $CO_2$. Further, 5 mg/ml MTT phosphate buffered saline (10 μl) was added and the incubation was continued for additional 4 hours. Cell growth was observed under a microscope and the apoptosis inducing activity was estimated as cell growth inhibitory activity.

The same procedure was repeated with respect to HL-60 cells except that the cells were cultured in RPMI1640 culture medium containing 10% fetal bovine serum.

As a result, with respect to NIH/3T3 cells, no cell growth was observed in the group to which the cyclopentenone had been added at a final concentration of 25 μM, while the same cell growth as that of the water-added control group was observed in the group to which the cyclopentenone has been added at a final concentration of 12.5 μM. To the contrary, with respect to HL-60 cells, no cell growth was observed in the group to which the cyclopentenone had been added at a final concentration of 6.25 μM, and almost all the cells were killed. Thus, it has been clarified that the cyclopentenone showed selective cell growth inhibitory activity and cytocidal activity against tumor cell line HL-60 as compared with non-tumor cell line NIH/3T3.

Example 7

Activity of Fucoidan-U on HUVEC and HL-60

HUVEC cells was passaged once and frozen for storage according to a conventional manner. The cells were thawed and, after washing twice with phosphate buffered saline, they were suspended in EBM culture medium (manufacture by Sanko Pure Chemical) containing 10% fetal bovine serum and 0.1% bovine brain extract at a concentration of $1 \times 10^5$ cells/ml. Each 900 μl portion of the cell suspension was distributed to each well of a 12-well microtiter plate and added thereto 100 μl of aqueous Fucoidan-U solution (10 mg/ml) or physiological saline (control). The cells on the plate were incubated at 37°C for 24 or 48 hours in the presence of 5% $CO_2$. After treatment with trypsin, the cells were collected. They were stained with trypan blue, and the living cell number and the dead cell number were counted to calculate the living cell ratio to determine apoptosis inducing activity.

The same procedure was carried out with respect to HL-60 cells except that the cells used was passaged in RPMI1640 culture medium containing 10% fetal bovine serum and no treatment with trypsin was carried out.

As a result, the group to which Fucoidan-U had been added to HUVEC cells at a final concentration of 1 mg/ml showed the same living cell ratio as that of the physiological saline-added control group, while the group to which Fucoidan-U had been added to HL-60 cells at a final concentration of 1 mg/ml showed an apparent decrease in the

living cell ratio in comparison with the physiological saline-added control group. Thus, it has been clarified that Fucoidan-U induces apoptosis specific to cancer cells to decrease their living cell ratio.

The results are shown in Figs. 10 and 11. Fig. 10 is the graph illustrating the relation between the incubation time and the living cell ratio of HUVEC cells, wherein the horizontal axis represents the incubation time (hrs.) and the vertical axis represents the living cell ratio (%). In Fig. 10, the filled circle represents the results obtained by using Fucoidan-U and the blank circle represents the results obtained by using the physiological saline added control. In Fig. 10, the living cell ratio at the initial stage when Fucoidan-U was added is almost the same as that when the physiological saline was added and the filled circle therefore is superimposed on the blank circles. Further, Fig. 11 is the graph illustrating the relation between the incubation time and the living cell ratio of HL-60 cells, wherein the horizontal axis represents the incubation time (hrs.) and the vertical axis represents the living cell ratio (%). In Fig. 11, the filled circle represents the results obtained by using Fucoidan-U and the blank circle represents the results obtained by using the physiological saline added control.

As described hereinabove, according to the present invention, there is provided the hematopoietic stem cell composition substantially free from cancer cells. When an exogenous gene is transferred into the composition and grafted into a host, hematopoietic stem cell grafting can be carried out in safe without such a risk that the gene is transferred into cancer cells. According to the present invention, cancer cells in a target cell composition for gene transfer can be eliminated to establish the safety of gene transfer.

BRIEF DESCRIPTION OF THE DRAWINGS

Fig. 1 is a graph illustrating precipitate formation of Fucoidan-U and -F.
Fig. 2 is a graph illustrating the relation between the incubation time and the living cell number in the presence of Fucoidan-U and -F.
Fig. 3 is an enlarged graph of Fig. 2, wherein the scale on the vertical axis is enlarged.
Fig. 4 is a graph illustrating the relation between the incubation time and the living cell number of HL-60 cells.
Fig. 5 is a graph illustrating the relation between the fucoidans and the high density colony number.
Fig. 6 is a graph illustrating the relation between the incubation time and the living cell number in the presence of the dextran sulfate.
Fig. 7 is a graph illustrating the relation between the incubation time and the living cell number in the presence of the heated dextran sulfate.
Fig. 8 is a graph illustrating the relation between the incubation time and the living cell number in the presence of the heat-treated pectin.
Fig. 9 is a graph illustrating the relation between the cyclopentenone concentration added and growth of cells.
Fig. 10 is a graph illustrating the relation between the incubation time and the living cell ratio of HUVEC cells.
Fig. 11 is a graph illustrating the relation between the incubation time and the living cell ratio of HL-60 cells.

## Claims

**1.** An in vitro process for obtaining a cell composition comprising hematopoietic stem cells from which cancer cells have been substantially eliminated, said process comprising the step for selectively eliminating cancer cells in said cell composition by treating it with an apoptosis inducer specific for cancer cells which is selected from the group consisting of a sulfated polysaccharide, a sulfated polysaccharide which is heat treated, enzymatically hydrolysed, treated with near infrared rays or with infrared rays, treated with ultrasonic waves or treated with microwave irradiation and having apoptosis inducing properties, a saccharide compound containing a uronic acid and/or a uronic acid derivative, a saccharide compound containing a uronic acid and/or a uronic acid derivative which is heat treated, enzymatically hydrolysed, treated with near infrared rays or with infrared rays, treated with ultrasonic waves or treated with microwave irradiation and having apoptosis inducing properties, and 4,5-dihydroxy-2-cyclopenten-1-one.

**2.** A process according to claim 1 wherein the sulfated polysaccharide is a fucoidan or a dextran sulfate.

**3.** A process according to claim 2 wherein the apoptosis inducer is obtained by heat treatment or the sulfated polysaccharide.

**4.** A process according to claim 2 wherein the apoptosis inducer is obtained by treatment of the fucoidan or a dextran

sulfate with Flavobacterium FERM BP-5402 or its decomposing enzyme or wherein the dextran sulfate is obtained by treatment with an acid or alkali.

5. A process according to any one of claims 1 to 4, wherein the hematopoietic stem cells in a further step are transfected with an exogenous gene.

**Patentansprüche**

1. *In vitro*-Verfahren zur Gewinnung einer Zellzusammensetzung, die hämatopoetische Stammzellen umfasst und aus der Krebszellen im Wesentlichen beseitigt wurden, wobei das Verfahren den Schritt der selektiven Beseitigung von Krebszellen in der Zellzusammensetzung durch deren Behandlung mit einem Krebszell-spezifischen Apoptose-Induktionsmittel umfasst, das ausgewählt ist aus der Gruppe bestehend aus einem sulfatierten Polysaccharid, einem sulfatierten Polysaccharid, das hitzebehandelt, enzymatisch hydrolysiert, mit Strahlung im nahen Infrarotbereich oder Infrarotbereich behandeit, mit Ultraschallwellen oder Mikrowellenstrahlung behandelt ist und Apoptose-induzierende Eigenschaften aufweist, einer Saccharidverbindung mit einer Uronsäure und/oder einem Uronsäurederivat, einer Saccharidverbindung mit einer Uronsäure und/oder einem Uronsäurederivat, die hitzebehandelt, enzymatisch hydrolysiert, mit Strahlung im nahen Infrarotbereich oder Infrarotbereich behandelt, mit Ultraschallwellen oder Mikrowellenstrahlung behandelt ist und Apoptose-induzierende Eigenschaften aufweist, und 4,5-Dihydroxy-2-cyclopenten-1-on.

2. Verfahren gemäß Anspruch 1, wobei das sulfatierte Polysaccharid ein Fucoidan oder ein Dextransulfat ist.

3. Verfahren gemäß Anspruch 2, wobei das Apoptose-Induktionsmittel durch Hitzebehandlung des sulfatierten Polysaccharids erhalten wird.

4. Verfahren gemäß Anspruch 2, wobei das Apoptose-Induktionsmittel durch Behandlung des Fucoidans oder eines Dextransulfats mit Flavobacterium FERM BP-5402 oder seinem Abbauenzym erhalten wird oder wobei das Dextransulfat durch Behandlung mit einer Säure oder Alkali erhalten wird.

5. Verfahren gemäß einem der Ansprüche 1 bis 4, wobei die hämatopoetischen Stammzellen in einem weiteren Schritt mit einem exogenen Gen transfiziert werden.

**Revendications**

1. Procédé *in vitro* pour obtenir une composition cellulaire comprenant des cellules souches hématopoïétiques à partir desquelles des cellules cancéreuses ont été sensiblement éliminées, ledit procédé comprenant l'étape consistant à éliminer sélectivement les cellules cancéreuses dans ladite composition cellulaire en la traitant avec un inducteur de l'apoptose spécifique des cellules cancéreuses qui est sélectionné dans le groupe constitué d'un polysaccharide sulfaté, d'un polysaccharide sulfaté qui est traité à la chaleur, hydrolysé enzymatiquement, traité avec des rayons de l'infrarouge proche ou avec des rayons infrarouges, traité avec des ondes ultrasoniques ou traité avec un rayonnement micro-onde et ayant des propriétés induisant l'apoptose, un composé saccharidique contenant un acide uronique et/ou un dérivé d'acide uronique, un composé saccharide contenant un acide uronique et/ou un dérivé d'acide uronique qui est traité à la chaleur, hydrolysé enzymatiquement, traité avec des rayons de l'infrarouge proche ou avec des rayons infrarouges, traité avec des ondes ultrasoniques ou traité avec un rayonnement micro-onde et ayant des propriétés induisant l'apoptose, et 4,5-dihydroxy-2-cylopentène-1-one.

2. Procédé selon la revendication 1, dans lequel le polysaccharide sulfaté est un fucoïdane ou un sulfate de dextran.

3. Procédé selon la revendication 2, dans lequel l'inducteur de l'apoptose est obtenu par traitement à la chaleur du polysaccharide sulfaté.

4. Procédé selon la revendication 2, dans lequel l'inducteur de l'apoptose est obtenu par traitement du fucoïdane ou d'un sulfate de dextran avec Flavobacterium FERM BP-5402 ou son enzyme de décomposition ou dans lequel le sulfate de dextran est obtenu par traitement avec un acide ou une base.

5. Procédé selon l'une quelconque des revendications 1 à 4, dans lequel les cellules souches hématopoïétiques sont

transfectées dans une étape ultérieure avec un gène exogène.

Fig. 1

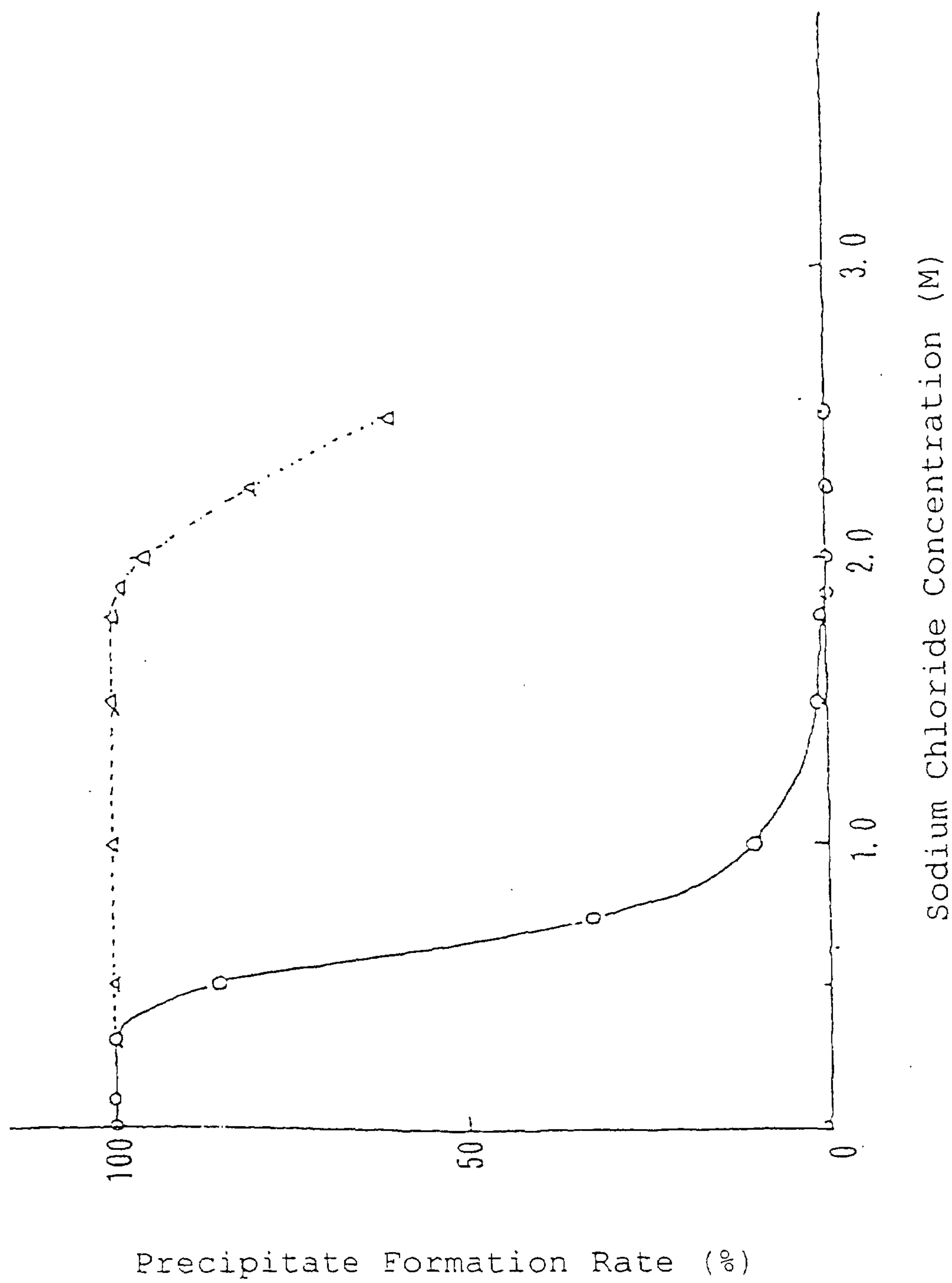
Sodium Chloride Concentration (M)
Precipitate Formation Rate (%)
0
50
100
1.0
2.0
3.0

Fig. 2

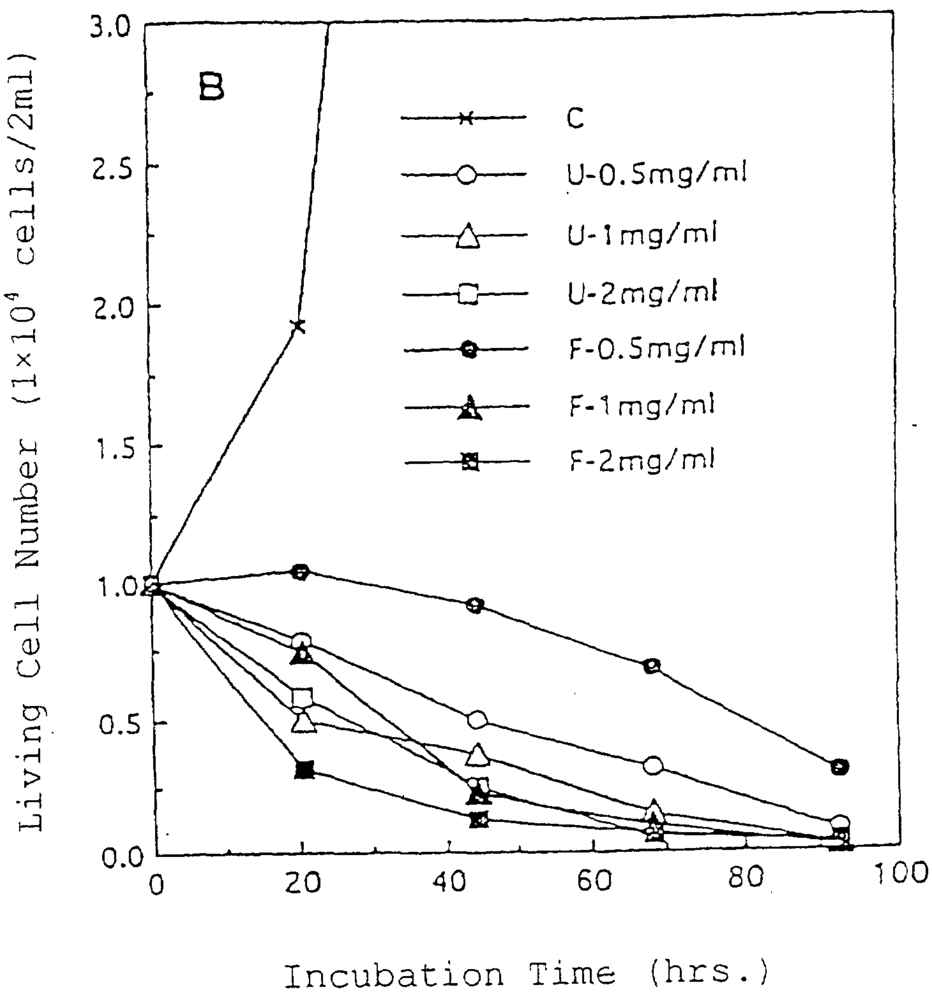
B
C
U-0.5mg/ml
U-1mg/ml
U-2mg/ml
F-0.5mg/ml
F-1mg/ml
F-2mg/ml
Living Cell Number (1×10⁴ cells/2ml)
3.0
2.5
2.0
1.5
1.0
0.5
0.0
0
20
40
60
80
100
Incubation Time (hrs.)

Fig. 3

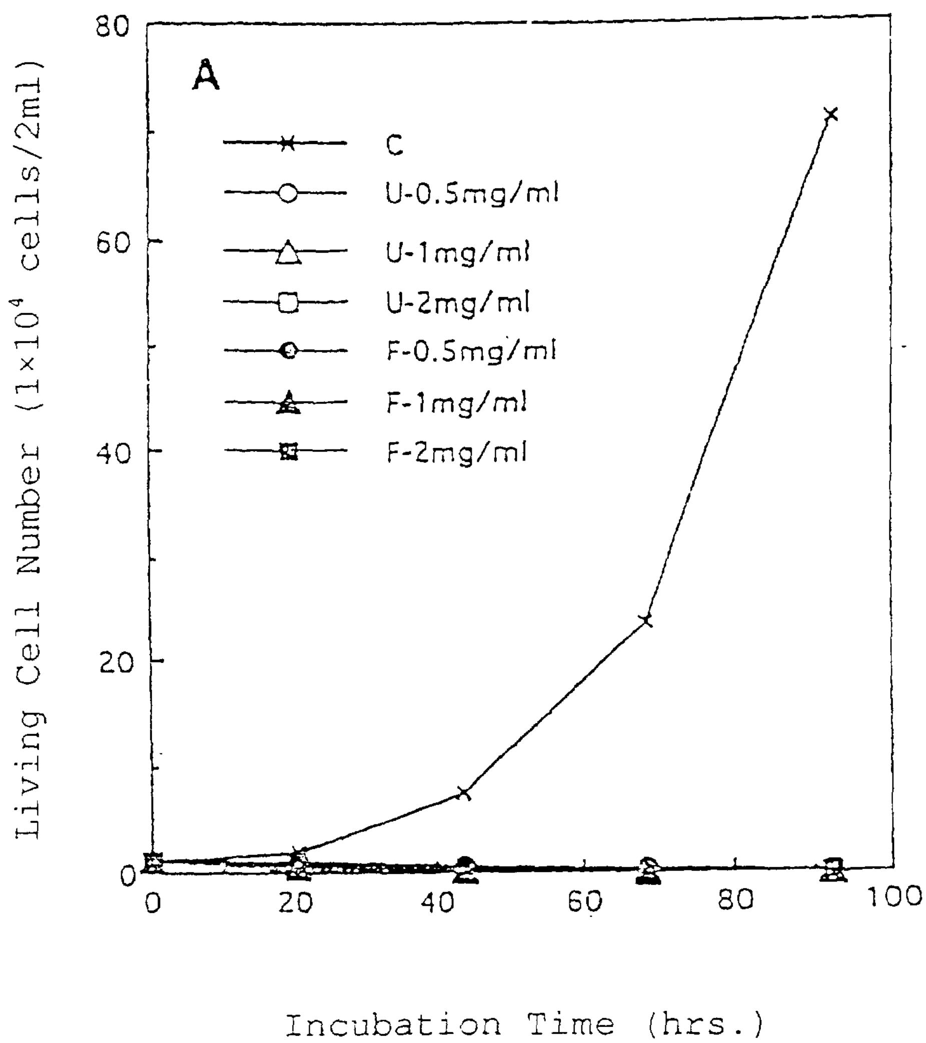
A
C
U-0.5mg/ml
U-1mg/ml
U-2mg/ml
F-0.5mg/ml
F-1mg/ml
F-2mg/ml
Living Cell Number (1×10⁴ cells/2ml)
0
20
40
60
80
0
20
40
60
80
100
Incubation Time (hrs.)

Fig. 4

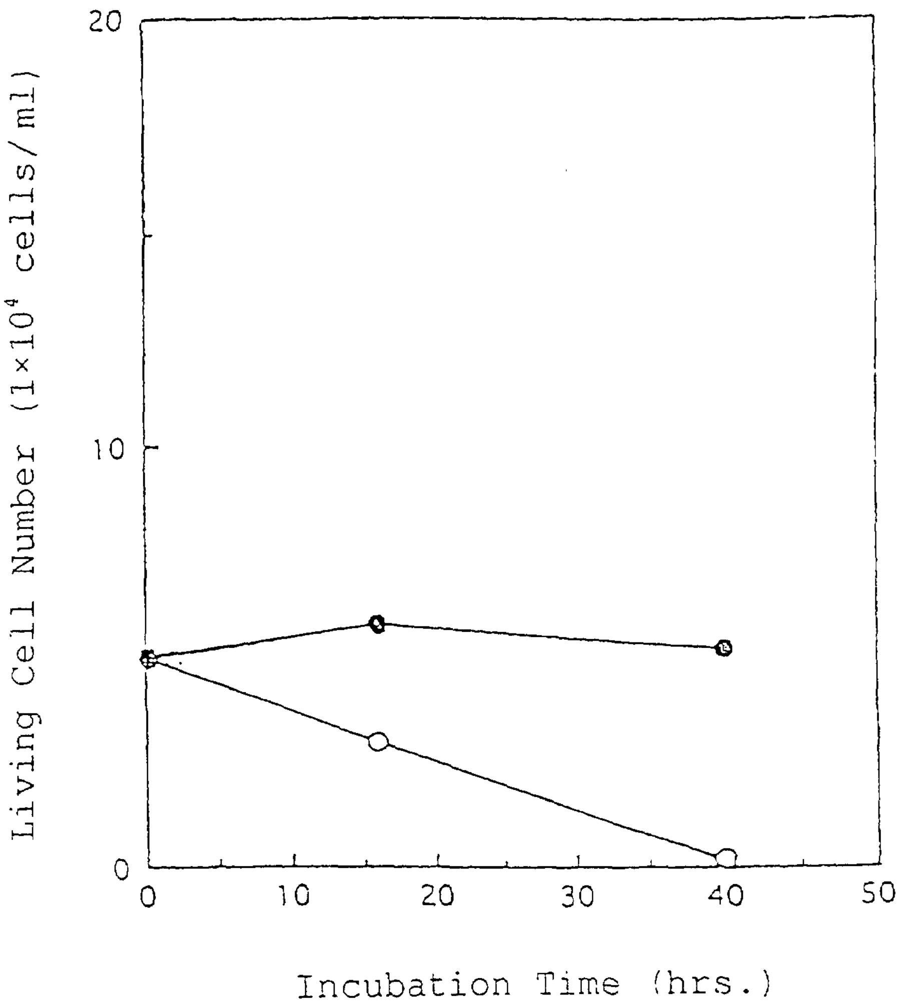
20
10
0
Living Cell Number $(1\times10^4$ cells/ml)
0
10
20
30
40
50
Incubation Time (hrs.)

Fig. 5

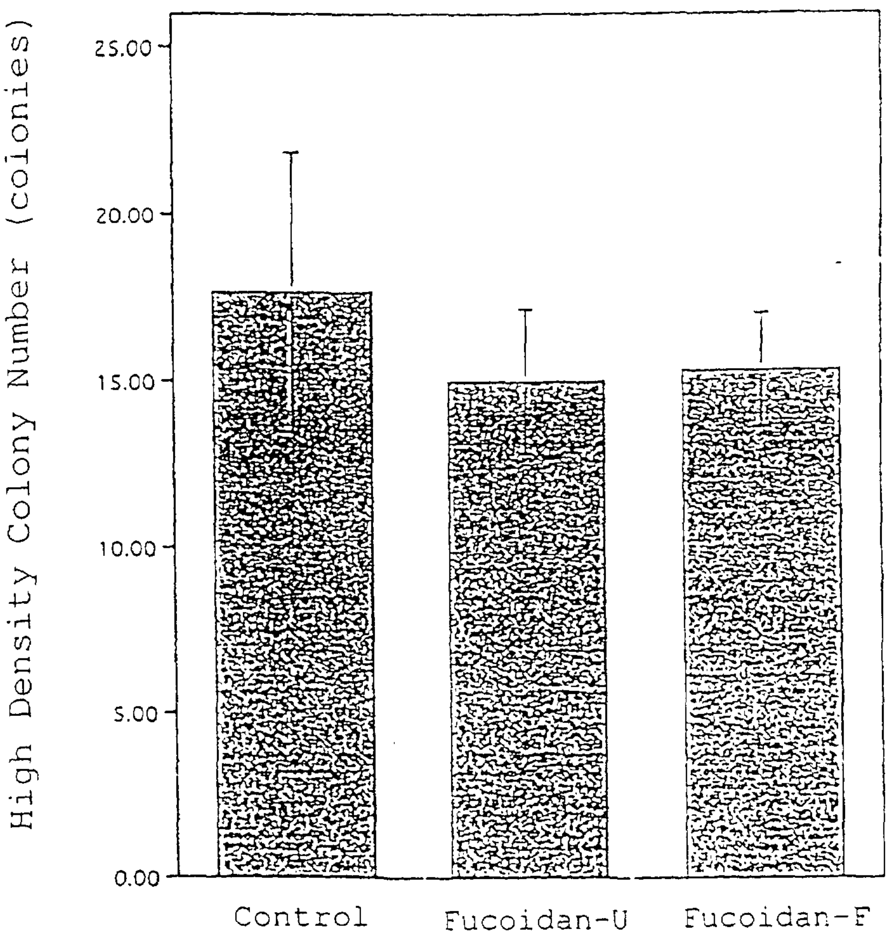
High Density Colony Number (colonies)
25.00
20.00
15.00
10.00
5.00
0.00
Control
Fucoidan-U
Fucoidan-F

Fig. 6

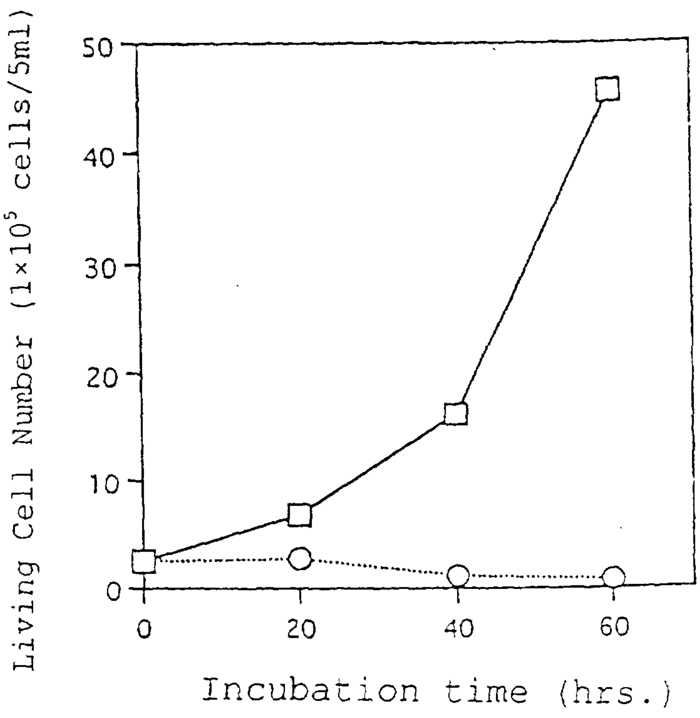
Living Cell Number ($1\times10^5$ cells/5ml)
50
40
30
20
10
0
0
20
40
60
Incubation time (hrs.)

Fig. 7

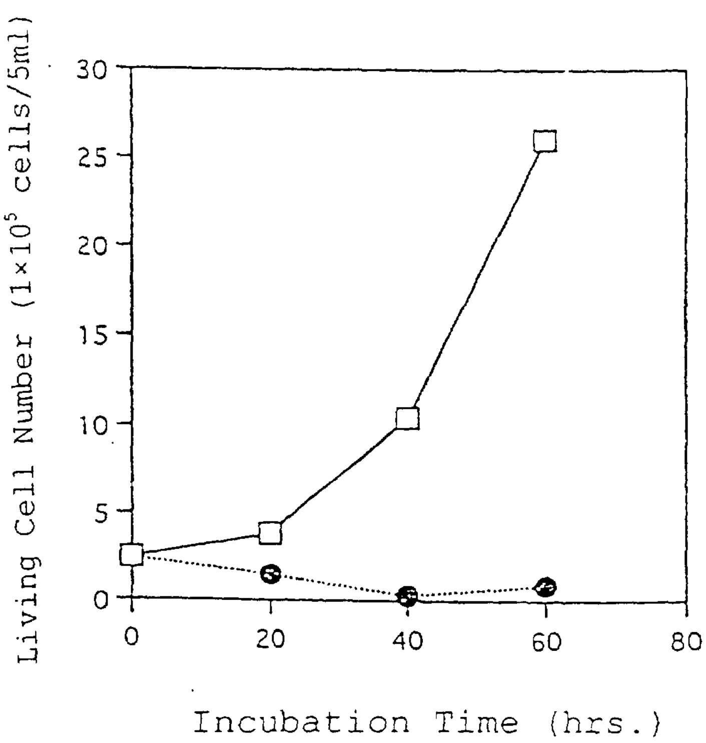
Living Cell Number ($1\times10^5$ cells/5ml)
30
25
20
15
10
5
0
0
20
40
60
80
Incubation Time (hrs.)

Fig. 8

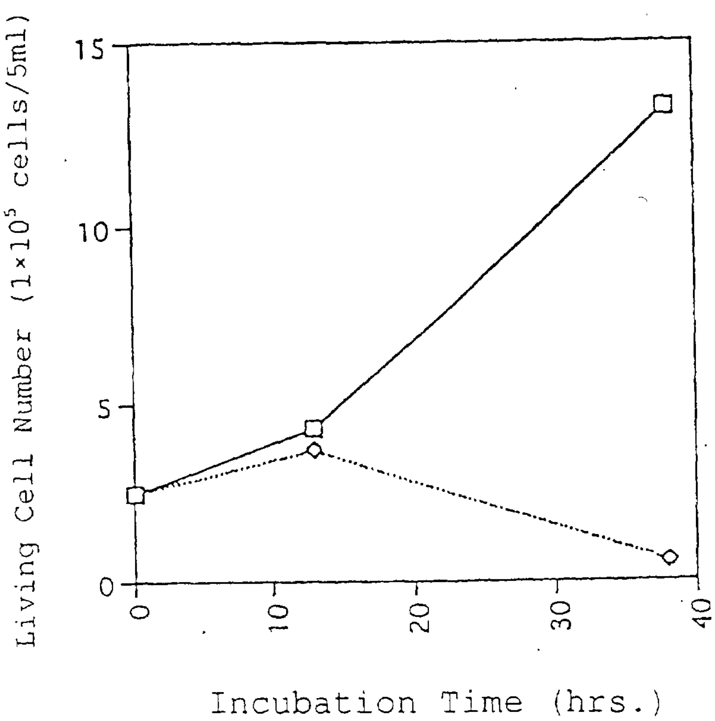
Living Cell Number (1×10⁵ cells/5ml)
15
10
5
0
0
10
20
30
40
Incubation Time (hrs.)

Fig. 9

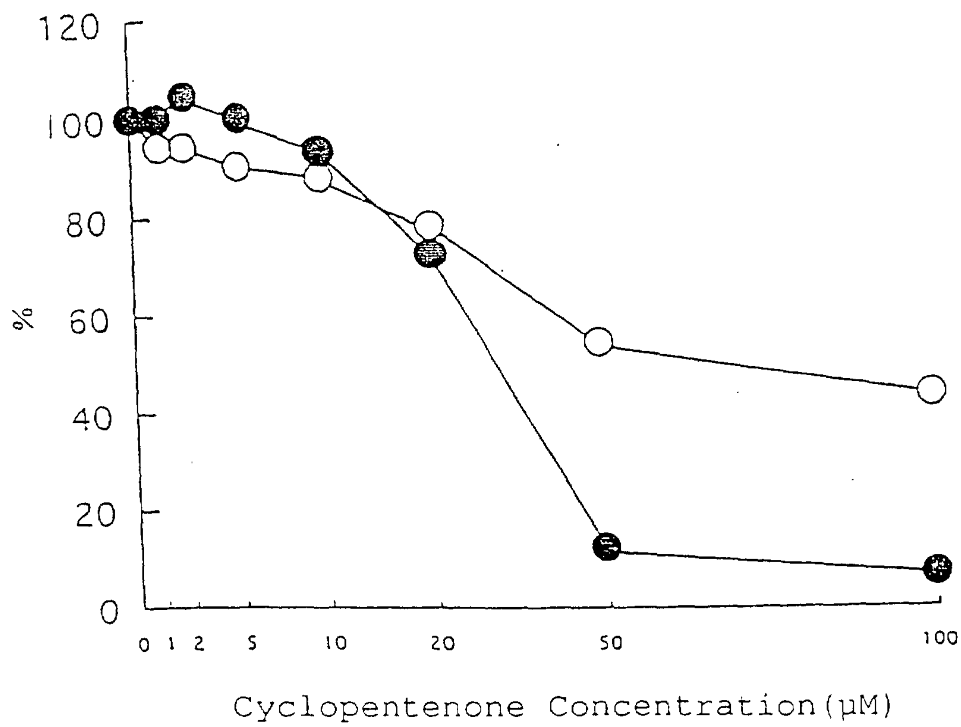
120
100
80
%
60
40
20
0
0 1 2 5 10 20 50 100
Cyclopentenone Concentration(μM)

Fig. 10

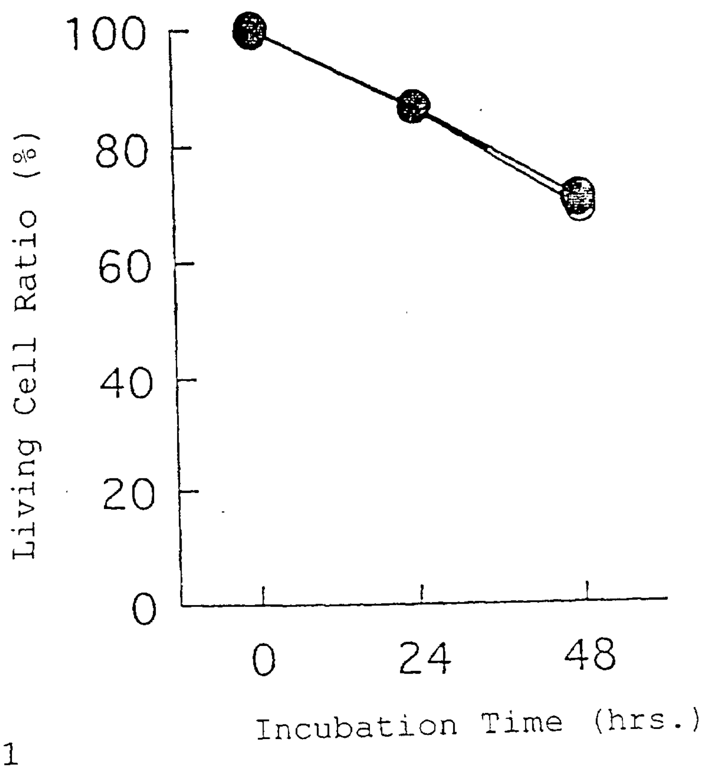
100
80
60
40
20
0
Living Cell Ratio (%)
0
24
48
Incubation Time (hrs.)

Fig. 11

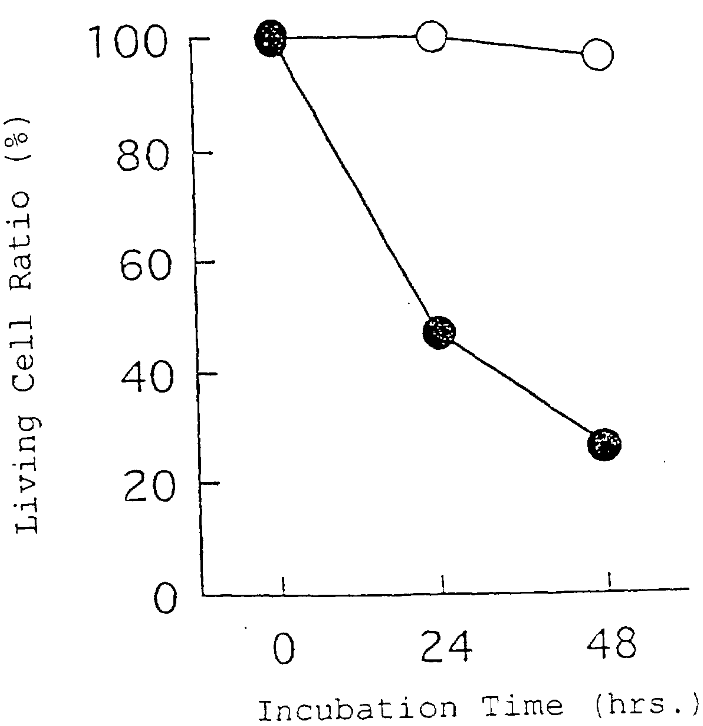
100
80
60
40
20
0
Living Cell Ratio (%)
0
24
48
Incubation Time (hrs.)